(19) Europäisches Patentamt
European Patent Office
Office européen des brevets

(11) Veröffentlichungsnummer : **0 436 682 B1**

# (12) EUROPÄISCHE PATENTSCHRIFT

(45) Veröffentlichungstag der Patentschrift : **19.01.94 Patentblatt 94/03**

(51) Int. Cl.$^5$ : **A61K 35/78,** A61K 31/575, A61K 31/70

(21) Anmeldenummer : **90909621.6**

(22) Anmeldetag : **06.07.90**

(86) Internationale Anmeldenummer : **PCT/CH90/00164**

(87) Internationale Veröffentlichungsnummer : **WO 91/01139 07.02.91 Gazette 91/04**

## (54) STEROLE, DEREN FETTSÄUREESTER UND GLUCOSIDE; VERFAHREN ZU IHRER HERSTELLUNG; SPONTAN DISPERGIERBARE MITTEL MIT DIESEN VERBINDUNGEN, SOWIE IHRE VERWENDUNG ZUR BEHANDLUNG VON TUMOREN.

(30) Priorität : **21.07.89 CH 2727/89**
**02.12.89 CH 4308/89**

(43) Veröffentlichungstag der Anmeldung : **17.07.91 Patentblatt 91/29**

(45) Bekanntmachung des Hinweises auf die Patenterteilung : **19.01.94 Patentblatt 94/03**

(84) Benannte Vertragsstaaten : **DE FR GB IT NL**

(56) Entgegenhaltungen :
**EP-A- 289 636**
**FR-A- 2 240 717**
**GB-A- 931 115**

(73) Patentinhaber : **MARIGEN S.A.**
**Eugster, Carl, Hackbergstrasse 40**
**CH-4125 Riehen (CH)**

(72) Erfinder : **EUGSTER, Carl**
**Hackbergstrasse 40**
**CH-4125 Riehen (CH)**
Erfinder : **EUGSTER, Conrad, Hans**
**Herrengütlistrasse 18**
**CH-8304 Wallisellen (CH)**
Erfinder : **HALDEMANN, Walter**
**Zeigerweg 23**
**CH-4102 Binningen (CH)**
Erfinder : **RIVARA, Giorgio**
**Le Molinette Corso Bramante, 88**
**I-10126 Torino (IT)**

(74) Vertreter : **Haldemann, Walter, Dr.**
**c/o MARIGEN S.A. 40, Hackbergstrasse**
**CH-4125 Riehen (CH)**

EP 0 436 682 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft Sterole, deren Fettsäureester und Glucoside; Verfahren zu ihrer Herstellung; spontan dispergierbare Mittel mit diesen Verbindungen, sowie ihre Verwendung zur Behandlung von Tumoren.

Es wurde überraschenderweise gefunden, dass hauptsächlich die aus den Extrakten der Samen der Sonnenblume (Helianthus annuus L.) und gewisser Kürbisarten (Cucurbita pepo L. und Cu-curbita maxima Duch.) gewonnenen Sterole, deren Glucoside und insbesondere deren Fettsäureester, wie auch die mit diesen Verbindungen hergestellten spontan dispergierbaren Mittel eine vorzügliche antitumorale Wirkung aufweisen.

In der Literatur werden keine Angaben gemacht, dass Sterole, deren Glucoside und insbesondere deren Fettsäureester zur Behandlung von Tumoren eingesetzt werden können.

Erfindungsgemäss zu verwendende Sterole, deren Fettsäureester - und Glucoside können z.B. aus vorzugsweise vorgekeimten Samen von Helianthus annuus L., Cucurbita pepo L. (varietas styriaca) oder Cucurbita maxima Duch. unter Einhaltung folgender Verfahrensvorschriften gewonnen werden:

Die gewaschenen und während 2 - 4 Tagen vorgekeimten Samen werden mit destilliertem Wasser versetzt, welches 0,1 - 3 %, vorzugsweise 0,1 - 1 % Mannit und 0,1 - 4 % eines pharmaverträglichen, nicht-ionogenen Tensides bzw. Tensidgemisches (bezogen auf das Gewicht der vorgekeimten Samen) enthält. Das Gemisch wird in einer Zahnkolloidmühle homogenisiert und das so gewonnene Homogenat anschliessend zentrifugiert. Die entstehenden drei Phasen trennt man voneinander. Die untere Phase mit den Schalenteilen und den Zelltrümmern wird verworfen. Die obere, oelige Phase wird nach einem Extraktionsschritt präpativ chromatographisch gereinigt, wogegen die mittlere, wassrige Phase zunächst ultrafiltriert, daran anschliessend im Vakuum eingeengt und als Konzentrat lyophilisiert wird.
(Vgl. dazu die Aufarbeitungsbeispiele 1 bis 3).

Die bevorzugt erfindungsgemäss zu verwendenden Fettsäureester von Sterolen lassen sich allgemein auch halbsynthetisch nach folgenden, an sich bekannten Verfahren herstellen:

a) Umsetzung der Fettsäure mit N, N'-Carbonyl-diimidazol bei 25 - 70°C unter Zusatz einer katalytischen Menge eines Alkoholates in Tetrahydrofuran, Benzol, Chloroform oder Dimethylformamid oder in einem ähnlichen indifferenten Lösungsmittel und anschliessender Alkoholyse der gebildeten Imidazolide mit einem Sterol, wie z.B.

24 β-Aethylcholesta - 5,22,25 - trien - 3β-ol (25,27 Dehydroporiferasterol)
24 β-Aethylcholesta - 5,25 (27) - dien - 3β-ol (Clerosterol)
24 z-Aethylidencholest - 5 - en - 3β-ol (Isofucosterol)
24 α-Aethylcholesta - 5,22 - dien -3β-ol (Δ5-Stigmasterol)
24 α-Aethylcholest - 5 - en - 3β-ol (Δ5-Sitosterol)
24 α-Aethylcholest - 7 - en - 3β-ol (Δ7-Sitosterol)
24 α-Aethyl - 5α - cholesta - 8,22 - dien - 3β-ol
24 β-Aethyl - 5α - cholesta - 8,55(27)-dien-3β-ol

b) Bildung des Chlorids der Fettsäuren mit Thionylchlorid in einem indifferenten Lösungsmittel und anschliessende Umsetzung des entstandenen Fettsäurechlorids mit einem Sterol {vgl. dazu Synthese a)}.

Für die Synthesen a) und b) eignen sich u.a. folgende geradkettigen und verzweigten, gesättigten und ungesättigten Fettsäuren:
Valeriansäure, Isovaleriansäure, Sorbinsäure, Isocapronsäure, Laurinsäure, Myristinsäure, Palmitinsäure, Stearinsäure, Arachidsäure, Behensäure, Hexacosansäure, Octacosansäure, Pentadecansäure, Heptadecansäure, Nonadecansäure, Tricosansäure, Pentacosansäure, Decenylsäure, Undecenylsäure, Dodecenylsäure, Oleinsäure, Linolsäure, Linolensäure, Arachidonsäure, Erucasäure, etc.

Die Glucoside der Sterole können nach einem an sich bekannten Verfahren in der Weise hergestellt werden, dass man in Gegenwart eines Katalyten wie z.B. Silbercarbonat ein Steroid wie beispielsweise Sitosterol, Stigmasterol oder Cholesterol mit Acetobromglucose in einem inerten Lösungsmittel wie z.B. Benzol, Toluol, Chloroform oder Tetrahydrofuran umsetzt.

Die nach Massgabe der Erfindung spontan dispergierbaren Konzentrate besitzen überraschenderweise eine gesteigerte, und hauptsächlich auch eine prophylaktische Antitumorwirkung. Sie ergeben, mit Wasser versetzt, Microemulsionen von ausgezeichneter Phasenstabilität und von erhöhtem Permeabilitäsvermögen.

Diese erfindungsgemässen, spontan dispergierbaren Konzentrate enthalten

0,001 bis 15 Gew.% einzelner Fettsäure-Ester von Sterolen bzw. Kombinationen dieser Komponenten
0 bis 40 Gew.% eines als Hydrotrop, bzw. Co-Emulgator dienenden, pharmaverträglichen Lösungsmittels oder Lösungsmittelgemisches
0,001 bis 85 Gew.% eines pharmaverträglichen Tensides oder Tensidgemisches
0 bis 10 Gew.% eines Vitamins oder Provitamins

0 bis 10 Gew.% einer freien Fettsäure
und gegebenenfalls übliche Trägerstoffe und/oder Verdünnungsmittel.

Die erfindungsgemäss zu verwendenden Fettsäureester von Sterolen haben folgende Formeln:

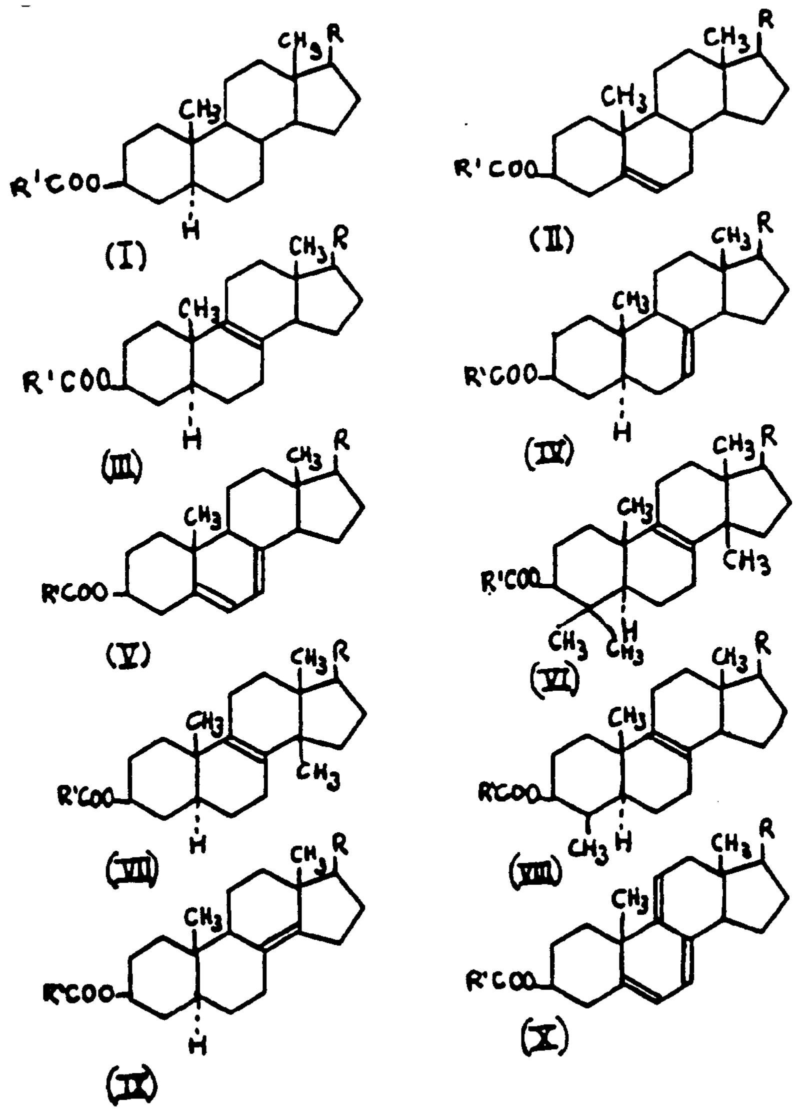

(I) (II) (III) (IV) (V) (VI) (VII) (VIII) (IX) (X)

In den Formeln I - X bedeuten R eine $C_1$ bis $C_{10}$-Alkylgruppe oder eine $C_2$ bis $C_{10}$-Alkenylgruppe und R' eine $C_1$ bis $C_{32}$ -Alkyl- oder eine $C_2$ bis $C_{32}$-Alkenyl- bzw. Alkapolyengruppe (d.h. die entsprechenden Alkadiene, Alkatriene, Alkatetraene, Alkapentaene oder Alkahexaene). Diese Seitenketten bei R und R' können geradkettig oder verzweigt sein.

Bei R haben die Alkyl- und die Alkenylgruppen vorzugsweise 8 - 10 Kohlenstoffatome. Beispiele solcher Verbindungen sind u.a.:

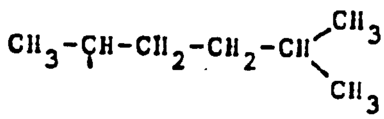

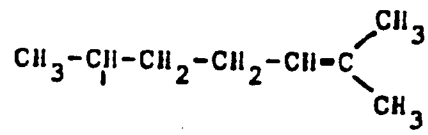

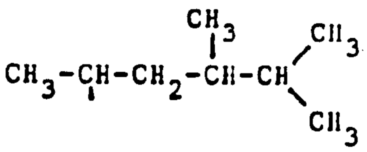

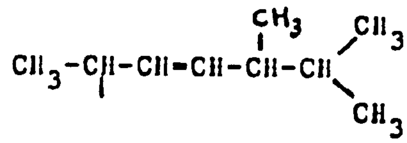

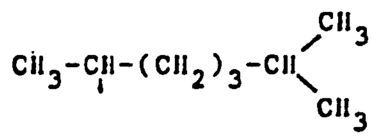

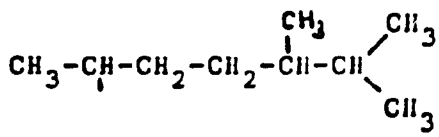

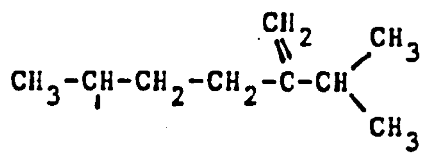

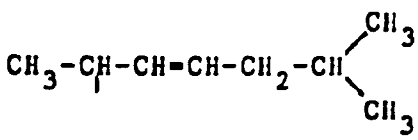

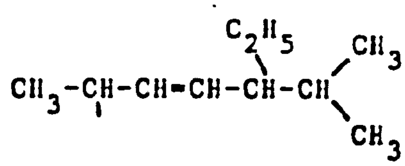

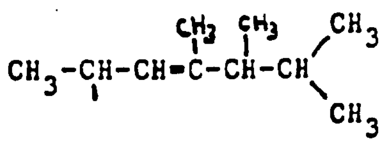

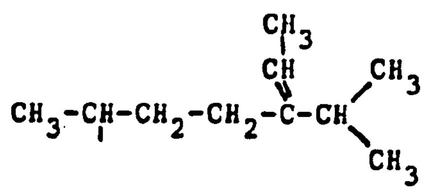

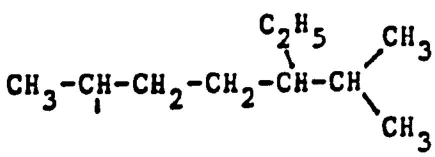

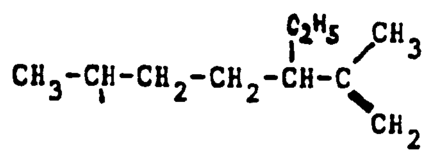

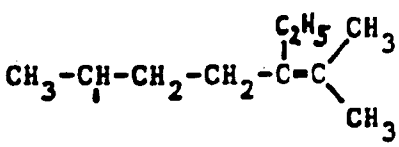

und

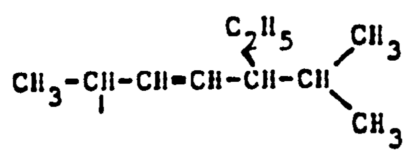

Bei R' haben die Alkyl- und Alkenyl-/Alkapolyengruppen (mit 1 bis 6 Doppelbindungen) vorzugsweise 8 bis 22 Kohlenstoffatome. Besonders bevorzugt sind bei R' Alkyl- und Alkenyl-/Alkapolyengruppen mit 10 bis 18 Kohlenstoffatomen.

Beispiele von erfindungsgemäss zu verwendenden Fettsäureestern von Sterolen der Formeln I bis X sind u.a.:

Ergosta-5,7-dien-3-ol-9-hexadecenoat
(Ergosta-5,7-dienylpalmitoleat)
Ergosta-8,22-dien-3-ol-14-methyl 4,9-octadecenoat
(14α-Methylergosta-8,22-dienyloleat)
Lanost-8-en-3-ol-9-octadecenoat
(Dihydrolanosterol-oleat)
Ergost-5-en-3-ol-9,12,15-octadecatrienoat
(Dihydrobrassicasteryl-linolenat)
Ergost-5-en-3-ol-9,12-octadecadienoat

Ergost-5-en-3-ol-9-octadecenoat
(Dihydrobrassicasteryl-oleat)
Ergosta-7,24 (28)-dien-3-ol-4-methyl-9-octadecenoat
(Gramisteryl-oleat)
Stigmosta-8,24 (28)-dien-3-ol-9,12-octadecadienoat
($\Delta^7$-Avenasteryl-linoleat)
Ergosta-7,24 (28)-dien-3-ol-4-methyl-9,12-octadecadienoat
(Gramisteryl-linoleat)
Stigmast-24 (28)-en-3-ol-9,12-octadecadienoat
Ergosta-5,22-dien-3-ol-4,23-dimethyl-9-octadecenoat
Ergostan-3-ol-4-methyl-9-octadecenoat
5α-Stigmastan-3β-ol-linolenat
5α-Stigmastan-3β-ol-oleat
Stigmastan-3-ol-9,12-octadecadienoat
(5α-Stigmastan-3β-ol-linoleat)
22-Dihydrospinasteryl-linoleat
Ergosta-5,7,22-trien-3-ol-9,12-octa-decadienoat
(Ergosterol-linoleat)
Stigmasta-5,24 (28)-dien-3-ol-9-octadecenoat
Stigmasta-5,24 (28)-3-ol-9,12-octadecadienoat
Stigmasta-5-en-3-ol-5,8,11,14-eicosatetraenoat
(β-Sitosterol-arachidonat)
Ergost-5-en-3-ol-5,8,11,14-Eicosatetraenoat
Stigmasta-7,24 (28)-dien-3-ol-4-methyl-9,12-octadecadienoat
Cholest-5-en-3-ol(3β)-9-hexadecenoat
(Cholesteryl-trans-9-hexadecenoat)
Ergost-7-en-3-ol-9,12,15-octadecatrienoat
Ergost-5-en-3-ol-9,12,15-octadecatrienoat
(Campesteryl-linolenat)
Ergostan-3-ol-9,12-octadecadienoat
Cholest-7-en-3-ol-9,12-octadecadienoat
Ergosta-5,24 (28)-dien-3-ol-9-hexadecenoat
Cholestan-3-ol-9-hexadecenoat
Ergosta-5,22-dien-3-ol-octadecenoat
(Brassicasteryl-oleat)
Cholest-7-en-3-ol-9-octadecenoat
(Lathosteryl-oleat)
Lanosta-8,24-dien-3-ol-9-octadecenoat
(Lanosterol-oleat)
Stigmasta-5,24(28)-dien-3-ol-9-octadecenoat
(Fucosteryl-oleat)
Cholesta-5,22-dien-3-ol-9-octadecenoat
(Desmosteryl-oleat)
Ergost-5-en-3-ol-12-octadecadienoat
(Campesteryl-linoleat)
Ergosta-5,22-dien-3-ol-9-octadecenoat
Ergost-22-en-3-ol-9-hexadecenoat
Ergosta-5,22-dien-3-ol-9-hexadecenoat
Cholesta-5,22-dien-3-ol-9-hexadecenoat
Ergosta-5,22-dien-3-ol-9,12-octadecadienoat
(Brassicasteryl-linoleat)
Ergosta-7,24(28)-dien-3-ol-9,12-octadecadienoat
Stigmasta-5,22-dien-3-ol-9,12,15-octadecatrienoat
(Stigmasterol-linoleat)
Stigmasta-5,22-dien-3-ol-9,12-octadecadienoat
(Stigmasterol-linoleat)
Cholest-5-en-3-ol- (3β)-5,8,11,14-eicosatetraenoat
Cholest-5-en-3-ol- (3β)-4,7,10,13,16,19-docosahexaenoat

Cholest-5-en-3-ol- (3β)-9,12-octadecadienoat
Cholesta-8,(14),24-dien-3-ol-9-octadecenoat
(Zymosteryl-oleat)
Ergost-5-en-3-ol-9-octadecenoat
(Campesteryl-oleat)
Cholesta-5,7,9 (11)-trien-3-ol-9-octadecenoat
(Cholesta-5,7,9 (11)-trien-3β-yl-oleat)
Ergosta-5,7,22-trien-3-ol-9-hexadecenoat
(Ergosterol-9-hexadecenoat)
Cholest-5-en-3-ol- (3β)-11-octadecenoat
(Cholesterol-11-octadecenoat)
Cholest-5-en-3-ol- (3β)-9,12-octadecadienoat
(Cholesterol-9,12-octadecadienoat)
Cholest-5-en-3-ol- (3β)-9-octadecenoat
(Cholesterol-elaidat)
5α-Stigmasta-7,22-dien-3β-ol-oleat
(α-Spinasterol-oleat)
Cholest-5-en-3-ol- (3β)-9-hexadecenoat
(Cholesterol-palmitoleat)
Cholestan-3-ol-9,12,15-octadecatrienoat
(Cholestanol-linoleat)
Cholest-5-en-3-ol- (3β)-11-octadecenoat
(Cholesterol-11-octadecenoat)
Cholesta-5,7-dien-3-ol-9-octadecenoat
Cholesta-5,7-dien- (3β)-ol-linoleat
(Cholecalciferol-linoleat)
Ergosta-5,7,22-trien-3-ol-9-octadecenoat
(Ergosterol-oleat)
Stigmast-5-en-3-ol-9-octadecenoat
(β-Sitosterol-oleat)
Stigmast-5-en-3-ol-9,12-octadecadienoat
(β-Sitosterol-linoleat)
Stigmast-5-en-3-ol-9,12,15-octadecatrienoat
(β-Sitosterol-linolenat)
Cholest-5-en-3-ol- (3β)-9,12,15-octadecatrienoat
(Cholesterol-linoleat)
Cholestan-3-ol-9-octadecenoat
(Cholestanol-oleat)
Cholestan-3-ol-9,12-octadecadienoat
(Cholestanol-linoleat)
Cholest-5-en-3-ol- (3β)-9-hexadecenoat
(Cholesterol-9-hexadecenoat)
Cholest-5-en-3-ol- (3β)-5,8,11,14-eicosatetraenoat
(Cholesterol-arachidonat)
Cholest-5-en-3-ol- (3β)-9,12-octadecadienoat
(Cholesterol-linoleat)
Cholest-5-en-3-ol- (3β)-9-octadecenoat
(Cholesterol-oleat)
β-Sitosterol-undecenoat
β-Sitosterol-lauroylat
β-Sitosterol-palmitat
Stigmasterol-undecenoat
Stigmasterol-lauroylat
Stigmasterol-palmitat

Die folgenden Beispiele von erfindungsgemäss zu verwendenden Fettsäureestern von Sterolen sind neu und bilden ebenfalls Teil der vorliegenden Erfindung:

γ-Sitostanol-oleat
γ-Sitostanol-linoleat

γ-Sitostanol-linolenat
γ-Sitosterol-oleat
Cholest-5-en-3α-ol-oleat
5-α-Stigmastan-3β-ol-oleat
5-α-Stigmastan-3β-ol-linolenat
Cholesta-5,7-dien-3β-ol-linoleat
Cholecalciferol-linolenat
10-α-Ergosta-5,7,22-trien-3β-ol-linoleat
Stigmast-5-en-3-ol-dodecenoat
(β-Sitosterol-dodecenoat)
Ergost-5-en-3-ol-dodecenoat
(Campesteryl-dodecenoat)
Cholest-7-en-3-ol-dodecenoat
Stigmasta-5,22-dien-3-ol-dodecenoat
(Stigmasterol-dodecenoat)
γ-Sitosterol-dodecenoat
Cholest-5-en-3-ol-undecenoat
Cholest-5-en-3-ol-dodecenoat
5-Cholesten-3β-ol-dodecenoat

Die erfindungsgemäss einzusetzenden Tenside oder Tensidgemische können anionaktiv, kationaktiv, amphoter oder nichtionogen sein. Am besten sind sie nicht-ionogen und haben einen HLB-Wert (d.h. eine "hydrophilic-lipophilic-balance") zwischen 2 - 18; bevorzugt liegt er zwischen 2 - 6 einerseits und 10 - 15 anderseits. HLB-Werte geben Auskunft über die lipophilen und hydrophilen Eigenschaften eines Emulgators. Vgl. dazu "Hydrophile - Lipophile Balance: History and recent Developments" von Paul Becher im Journal of Dispersion Science and Technology. 5 (1), 81-96 (1984).

Geeignete anionische Tenside können sowohl sog. wasserlösliche Seifen als auch wasserlösliche synthetische Verbindungen sein.

Als Seifen eignen sich die Alkali-, Erdalkali- oder gegebenenfalls substituierten Ammoniumsalze von höheren Fettsäuren ($C_{10}$ bis $C_{22}$), wie z.B. die natürlichen Na- oder K-Salze der Oel- oder Stearinsäure, oder von natürlichen Fettsäuregemischen, welche sich u.a. aus Kokosnuss- oder Talgöl gewinnen lassen. Ferner sind als Tenside auch die Fettsäure-Methyltaurinsalze, sowie modifizierte und nicht-modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate und -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst. Beispiele hiefür sind das Na- oder Ca-Salz der Ligninsulfosäure, des Dodecylschwefelsäureesters und Sulfonsäuren von Fettalkohol-Aethylenoxyd-Addukten. Die sulfonierten Benzimidazol-Derivate enthalten vorzugsweise zwei Sulfonsäuregruppen und einen Fettsäurerest mit etwa 8 - 22 C-Atomen. Alkylaryl-Sulfonate sind z.B. die Na-, Ca- oder Triäthanolaminsalze der Dodecylbenzolsulfonsäure, der Dibutylnaphthalinsulfonsäure oder eines NaphthalinsulfonsäureFormaldehydkondensationsproduktes. Ferner kommen auch entsprechende Phosphate, wie z.B. Salze des Phosphorsäureesters eines p-Nonylphenol-(4-14)-Aethylenoxyd-Adduktes, bzw. eines Adduktes der Formel

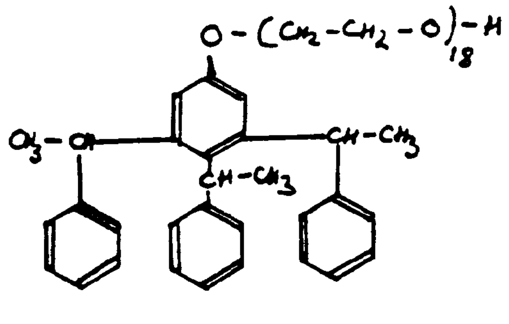

in Frage.

Als nichtionische Tenside stehen in erster Linie zur Wahl die Polyglykolätherderivate von aliphatischen oder cycloaliphatischen Alkoholen, gesättigten oder ungesättigten Fettsäuren und Alkylphenolen, welche 3 bis

30 Glykoläthergruppen und 8 bis 20 C-Atome im (aliphatischen) Kohlenwasserstoffrest und 6 bis 18 C-Atome im Alkylrest enthalten können. Weiterhin kommen als nichtionische Tenside in Frage die wasserlöslichen, 20 bis 250 Aethylenglykoläthergruppen und 10 bis 100 Propylenäthergruppen enthaltenden Polyäthylenoxydaddukte an Polypropylenglykol und Alkylpolypropylenglykol mit 1 bis 10 C-Atomen in der Alkylkette. Die genannten Verbindungen enthalten üblicherweise pro Propylenglykol-Einheit 1 bis 5 Aethylenglykoleinheiten.

Als Beispiele nicht-ionischer Tenside seien erwähnt:
Nonylphenolpolyäthoxyäthanole, Ricinusölpolyglykoläther, Polypropylen-Polyäthylenoxyd-Addukte, Tributylphenoxy-polyäthoxyäthanol, Polyäthylenglykol und Octylphenoxypolyäthoxyäthanol. Ueberdies kommen auch Fettsäureester von Polyoxyäthylensorbitan, wie das Polyoxyäthylensorbitantrioleat, in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorab als Halogenide, Methylsulfate oder Aethylsulfate vor, z.B. das Stearyltrimethylammoniumchlorid oder das Benzyl-di-(2-chloräthyl)-äthylammoniumbromid.

Als Hydrotrop, bzw. Co-Emulgator dienende, pharmaverträgliche Lösungsmittel lassen sich einsetzen, z.B.:
Ester eines aliphatischen Alkohols ($C_3$ - $C_{18}$) mit einer aliphatischen Carbonsäure ($C_{10}$ - $C_{22}$), wie etwa Isopropyllaurat, Hexyllaurat, Decyllaurat, Isopropylmyristat und Laurylmyristat; Kohlenwasserstoffe mit einer geraden Kohlenstoffkette ($C_{12-32}$), welche mit 6 - 16 Methylgruppen substituiert ist und bis 6 Doppelbindungen aufweisen kann, wofür Terpene wie Polymethylbutane und Polymethylbutene als Beispiele dienen mögen.

Mono-Ester aus Aethylenglykol oder Propylenglykol mit einer aliphatischen Carbonsäure ($C_6$ - $C_{22}$), wie etwa Propylenglykolmonolaurat und Propylenglykolmonomyristat.

Ester aus einem aliphatischen Alkohol ($C_{12}$ - $C_{22}$) mit Milchsäure, wie z.B. Myristyl- oder vorzugsweise Lauryl-Lactat. Mono- oder Diester des Glycerins mit einer aliphatischen Carbonsäure ($C_6$ - $C_{22}$), wie z.B. Glyceryl-Caprylat.

Ester aus einem Poly(2 - 7)äthylenglykolglyzerinäther mit mindestens einer freien Hydroxylgruppe mit einer aliphatischen Carbonsäure ($C_6$ - $C_{22}$), wie z.B. aliphatische Alkohole ($C_{12-22}$), somit u.a. Dodecanol, Tetradecanol, Oleylalkohol, 2-Hexyldecanol und 2-Octyl-decanol.
Ester mit mindestens einer freien Hydroxylgruppe, aus Poly-(2-10)glykol mit einer aliphatischen Carbonsäure ($C_6$ - $C_{22}$), Monoäther aus einem Polyäthylenglykol mit einem aliphatischen Alkohol ($C_{12-18}$), wie z.B. Polyoxyäthylen-($C_{10}$)octyläther.

Als Zusätze in die erfindungsgemässen spontan dispergierbaren Konzentrate eignen sich Vitamine und Provitamine (wie z.B. Vitamin A, Retinsäure, Retinol, Carotine, Tocopherole), sowie auch freie Fettsäuren wie etwa:
Valeriansäure, Isovaleriansäure, Sorbinsäure, Isocapronsäure, Laurinsäure, Myristinsäure, Palmitinsäure, Stearinsäure, Arachidsäure, Behensäure, Hexacosansäure, Octacosansäure, Pentadecansäure, Decenylsäure, Undecenylsäure, Dodecenylsäure, Oleinsäure, Linolsäure, Linolensäure, Arachidonsäure, Erucasäure, etc.

Die zur pharmazeutischen Anwendung erforderliche Tagesdosis beträgt 0,001 bis 25 mg/kg Körpergewicht, wenn möglich verteilt auf 2 - 3 Einzeldosen. Hiefür lassen sich die Fettsäureester von Sterolen oder die spontan dispergierbaren Konzentrate mit diesen Estern in die gängigen pharmazeutischen Zubereitungen und Darreichungsformen wie Dragées, Tabletten, Kapseln, Pulver, Granulat, Pellets, Lösungen, Ampullen, Emulsionen, Crèmes oder Zäpfchen zusammen mit den üblichen Trägerstoffen und/oder Verdünnungs- und Stabilisierungsmitteln einarbeiten.

Die Gegenstand der Erfindung bildenden Wirkstoffe oder Wirktoffmischungen, sowie die spontan dispergierbaren Konzentrate, welche diese Wirkstoffe oder Wirkstoffmischungen enthalten, können dem Menschen oral, durch Injektion (intravenös, subkutan oder intramuskulär) oder in anderer Weise verabreicht werden. Wenn sie als feste Darreichungsformen für die orale Verwendung aufbereitet werden, kann dies in Form von Tabletten, Granulaten, Pellets, Pulvern oder Kapseln, usw. geschehen. Die Aufbereitungen können Zusatzstoffe enthalten, z.B. einen Arzneimittelträger wie eine Saccharid- oder Cellulose-Grundlage, ein Bindemittel wie Stärkepaste oder Methyl-Cellulose, ein Füllmittel oder ein Desintegriermittel, usw. - wobei Zusatzstoffe eingesetzt werden, wie sie üblicherweise bei der Herstellung medizinischer oder paramedizinischer Formulierungen verwendet werden. Wenn die erfindungskonformen Wirkstoffe oder Wirkstoffmischungen als flüssige Darreichungsformen zu oraler Verabreichung gelangen, so können sie irgend eine aus wässrigen Zubereitungen für innere Verwendung, aus Suspensionen, Emulsionen und Sirups usw. ausgewählte Form haben, und sie können ausserdem in der Form getrockneter Präparate vorliegen, welche vor ihrer Verwendung in Lösung oder Emulsion gebracht werden.

Wenn die erfindungsgemässen Wirkstoffe oder Wirkstoffmischungen in der Form wässriger Lösungen, Suspensionen oder öliger, bzw. wässriger Emulsionen, vorzugsweise als Mikroemulsionen aus den erfindungskonformen, spontan dispergierbaren Konzentraten aufbereitet sind, können sie auch injiziert werden. Die Injektionslösungen werden jedoch üblicherweise kurz vor der Anwendung hergestellt, indem man die Extrakte oder Konzentrate in wässrigen, flüssigen Medien wie sterilem Wasser oder Physiologischer Kochsalzlösung auflöst oder suspendiert.

Falls erforderlich, können zu einem Injektionspräparat üblicherweise verwendete Lösungsmittel, Stabilisierungsmittel, Konservierungsmittel und Zusätze für die Herstellung isotonischer Lösungen hinzugegeben werden. Die auf diese Weise erhaltenen Injektions-Präparate werden intravenös, intramuskulär, subkutan oder in einer anderen geeigneten Weise verabreicht.

Die vorliegende Erfindung betrifft ebenfalls pharmazeutische Präparate, welche die Wirkstoffe, bzw. Wirkstoffgemische, bzw. die beschriebenen, spontan dispergierbaren Konzentrate zur Bekämpfung des Wachstums von Tumorzellen enthalten. Bei den der Erfindung entsprechenden pharmazeutischen Präparaten handelt es sich um solche zur enteralen (wie oralen oder rektalen), sowie zur parenteralen oder topischen Verabreichung an Warmblüter, welche das spontan dispergierbare Konzentrat allein oder zusammen mit einem pharmazeutisch anwendbaren Trägermaterial enthalten.

Die Dosierung der erfindungsgemässen Konzentrate hangt von der Warmblüterspezies, dem Alter und dem individuellen Zustand, sowie von der Verabreichungsart ab. So werden z.B. zur Erzielung eines Abtötungseffektes von Tumorzellen an Warmblütern mit geringem Körpergewicht, wie z.B. Mäusen, Ratten und Hamstern, bei sukutaner Verabreichung Dosen im Bereich von ca. 0,1 - 50 mg/kg Körpergewicht, und bei intraperitonealer Verabreichung Dosen im Bereich von 0,05 - 5 mg/kg Körpergewicht angewandt.

Die oralen und rektalen Formen der neuen pharmazeutischen Präparate enthalten zwischen 1 - 95 %, vorzugsweise zwischen 10 - 95 %, insbesondere zwischen 20 - 95 % des erfindungsgemässen, spontan dispergierbaren Konzentrates. Sie können z.B. in Dosis-Einheitsform vorliegen, also als Dragées, Micropellets, Tabletten, Suppositorien oder Ampullen und vor allem als Kapseln.

Geeignete pharmazeutisch anwendbare Trägerstoffe für die oralen Formen sind hauptsächlich Füllstoffe, wie Zucker (z.B. Lactose, Saccharose, Mannit oder Sorbit), Cellulosepräparate und/oder Calciumphosphate (z.B. Tricalcium- oder Calciumhydrogenphosphat), ferner Bindemittel wie Stärkekleister unter Verwendung von u.a. Mais-, Weizen-, Reis- oder Kartoffelstärke, Gelatine, Traganth, Methylcellulose, Hydroxyl-Methylcellulose, Natriumcarboxymethylcellulose und/oder Polyvinylpyrrolidon und/oder Sprengmittel (wenn erwünscht), wie die obgenannten Stärken, ferner Carboxymethylstärke, quervernetztes Polyvinylpyrrolidon, Agar, Alginsäure oder ein Salz davon, wie z.B. Natriumalginat.

Als Fliessreguliermittel sind z.B. die Polyäthylenglykole Nr. 200 - 600 und höher geeignet.

Die beim Menschen als Darreichungsform bevorzugten Gelatinekapseln werden mit geeigneten Ueberzügen versehen, wobei man u.a. konzentrierte Zuckerlösungen - welche gegebenenfalls arabischen Gummi, Talk, Polyvinylpyyrolidon, Polyäthylenglykol und/oder Titandioxid enthalten - Lacklösungen (wässrige oder solche, die unter Verwendung organischer Lösungsmittel aufbereitet worden sind), oder magensaft-resistente Ueberzüge aus Lösungen von geeigneten Cellulosepräparaten, wie mikrokristalliner Cellulose (Avicel), Acetylcellulosephtalat, Hydroxymethylcellulosephtalat oder einem Copolymerisat wie Eudragite® L 30 D verwendet.

Als erfindungsgemäss besonders geeignete, oral anwendbare pharmazeutische Darreichungsform eignen sich Steck-Kapseln aus Gelatine und einem Weichmacher, wie Glyzerin oder Sorbitol. Die Weich- bzw. Hartgelatine-Kapseln können das erfindungsgemässe, spontan dispergierbare Konzentrat im Gemisch mit Füllstoffen, wie Laktose, Bindemitteln wie Stärke und/ oder Gleitmitteln wie Talk oder Magnesium-Stearat und gegebenenfalls mit Stabilisatoren und Antioydantien wie z.B. $\alpha$-Tocopherol enthalten. Der Einsatz von geeigneten Flüssigkeiten wie flüssigen Polyäthylenglykolen No. 200 - 600 als Verdünnungsmittel kann sich empfehlen, wobei sich ebenfalls Stabilisatoren und Antioxydantien zufügen lassen.

Zur parenteralen Verabreichung werden die erfindungskonformen Konzentrate mit destilliertem Wasser versetzt. Der entstehenden wässrigen Injektions-Mikroemulsion können viskositätserhöhende Stoffe, wie z.B. Na-Carboxymethylcellulose, Sorbit, Mannit und/oder Dextran, und gegebenenfalls auch Stabilisatoren und Antioxydantien zugefügt werden.

Die pharmazeutischen Präparate für die parenterale Anwendung enthalten vorzugsweise zwischen 0,1 bis 60 %, vorab zwischen 1 bis 40 % des erfindungsgemässen, spontan dispergierbaren Konzentrates.

Als topisch anwendbare Präparate, welche sich insbesondere zur Prophylaxe von Hautkrebsarten eignen, kommen z.B. Cremen, Salben, Pasten, Schäume, Tinkturen und Lösungen in Betracht, welche zwischen 0,01 bis 70 % des erfindungsgemässen Konzentrates enthalten.

Für Crèmen und Oel-in-Wasser-Emulsionen, welche mehr als 50 % Wasser aufweisen, verwendet man als oelige Grundlage in erster Linie Fettalkohole, z.B. Lauryl-, Cetyl- oder Stearylalkohol, flüssige bis feste

Wachse, z.B. Isopropylmyristat, Woll- oder Bienenwachs und/oder Kohlenwasserstoffe wie z.B. Vaseline (Petrolatum) oder Paraffinoel. Zur Emulgierung dieser öligen Grundlagen eignen sich in erster Linie oberflächenaktive, pharmaverträgliche Substanzen mit vorwiegend hydrophilen Eigenschaften, wie z.B. nichtionogene Emulgatoren, vorab Fettsäureester von Polyalkoholen oder Aethylenoxydaddukten (etwa Polyglycerinfettsäureester oder Polyäthylensorbitan-Fettsäureester) mit einem HLB-Wert von unter 8. Zusätze zur Wasserphase sind u.a. Mittel, die die Austrocknung der Crèmen vermindern, z.B. Polyalkohole wie Glyzerin, Sorbit, Propylenglykol und/oder Polyäthylenglykole No. 200 - 600, ferner Konservierungsmittel, Riechstoffe etc.

Salben sind Wasser-in-Oel Emulsionen, die bis zu 70 %, vorzugsweise jedoch zwischen 20 und 50 % Wasser oder wässrige Phasen enthalten.

Als Fettphase kommen in erster Linie Kohlenwasserstoffe, z.B. Vaselin, Paraffinoel und/oder Hartparaffine in Betracht, welche zur Verbesserung des Wasserbindungsvermögens geeignete Hydroxydverbindungen, wie z.B. Fettalkohole oder Ester, davon etwa Cetylalkohol oder Wollwachsalkohole, enthalten.

Fallweise werden noch Emulgatoren mit einem HLB-Wert von 8 bis 16, wie z.B. Sorbitan-Fettsäureester (etwa Sorbitanisostearol) zugesetzt. Zusätze zur Wasserphase sind u.a. Feuchthaltungsmittel, wie Polyalkohole (Glycerin, Propylenglykol, Sorbit und/oder Polyäthylenglykole No. 200, 400, 600); ferner Konservierungsmittel, Riechstoffe, etc.

Fettsalben sind wasserfrei und enthalten als Grundlage vornehmlich Kohlenwasserstoffe, z.B. Paraffin, Vaselin und/oder flüssige Paraffine; überdies natürliche oder partial synthetische Fette wie z.B. Kokosfettsäuretriglycerid, ferner: Fettsäurepartialester des Glycerins, wie z.B. die im Zusammenhang mit den Salben erwähnten, die Wasseraufnahmefähigkeit steigernden Fettalkohole, Emulgatoren und/oder Zusätze.

Pasten sind Crèmen und Salben mit sekretabsorbierenden Puderbestandteilen, wie beispielsweise Metalloxide (etwa Titanoxid oder Zinkoxid), ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende Oel-in-Wasser Emulsionen der erfindungsgemässen, spontan dispergierbaren Konzentrate, wobei halogenierte Kohlenwasserstoffe (wie z.B. Chlorfluorniederalkane: etwa Dichlordifluormethan und Dichlortetrafluoräthan) als Treibmittel verwendet werden. Dazu kommen gegebenenfalls die üblichen Zusätze wie Konservierungsmittel, usw.

Gegenstand der vorliegenden Erfindung ist auch die Verwendung der erfindungsgemässen Wirkstoffe, Wirkstoffgemische, sowie der spontan emulgierbaren Konzentrate zur Hemmung des Wachstums von Tumorzellen oder als Prophylaktika gegen Tumorerkrankungen bei Mensch und Tier, wobei sie vorzugsweise in Darreichungsformen entsprechend den oben beschriebenen pharmazeutischen Präparaten verabreicht werden. Für den Einsatz als Diätetika und als Lebensmittelzusätze sind die optimalen Zusammensetzungen im Einzelnen fallweise zu bestimmen.

## AUFARBEITUNGSBEISPIELE

### 1. Gewinnung des lipophilen Anteils und des wässrigen Extraktes

750 g von den Schalen befreite Samen von Helianthus annuus L. (Sonnenblumenkerne) werden mit destilliertem Wasser befeuchtet und bei 30°C und 90 % relativer Luftfeuchtigkeit während 48 Stunden gelagert. Die gequollenen und angekeimten Samenkerne mit einem Gewicht von nun 1500 g versetzt man mit 1500 ml dest. Wasser, 20 g Mannit und 15 g Invadin® JFC 800 % (CIBA-GEIGY AG) und homogenisiert das Gemisch mit einem Waring Blendor und einer Zahnkolloid-Mühle der Firma Fryma AG., Rheinfelden. Das Homogenat von 3 kg wird in einer Suprafuge 22 (Heräus AG. Zürich) während einer Stunde bei +4°C mit 10'000 rpm = 18'100 g zentrifugiert. Es bilden sich drei Phasen. Die obere lipophile Phase wird abgenommen und die wässrige mittlere Phase von der unteren Phase mit den schwereren Fettstoffen und Wachsen, sowie den Zelltrümmern abdekantiert.

### 2. Aufarbeitung und Analyse des lipophilen Anteils.

200 g lipophile Phase aus dem Aufarbeitungsbeispiel 1 werden in 400 ml t-Butylmethyläther gelöst; die Lösung wird durch Filtration mit Papierfiltern von den Trübstoffen befreit. Aus dem Filtrat wird anschliessend das Lösungsmittel am Rotavapor (Büchi Laboratoriums-Technik AG, Flawil) im Vakuum von 5 mm/Hg (Membranpumpe) und bei einem Temperaturgefälle von 35°C auf 3,5°C abgedampft. Das zurückbleibende Oel wird durch präparative Chromatographie weiter gereinigt.

Die Analyse des gereinigten Oeles von Helianthus annuus L. gemäss Aufarbeitungsbeispiel 2 mittels Gaschromatographie ergibt folgende Werte:

| | |
|---|---|
| Freie Fettsäure | 1,07 % |

| | |
|---|---|
| Brechungsindex ($n_D$ 50°C) | 1,4649 |
| Peroxidzahl | 5,4 |

Wichtigste Fettsäureverteilung in % :

| | |
|---|---|
| C 16 | 5,6 |
| C 18 | 3,9 |
| C 18 : 1 | 16,0 |
| C 18 : 2 / Spur C 20 | 73,0 |
| C 20 : 1 | 0,2 |
| C 22 | 0,7 |

Der Gehalt an Tocopherol beträgt:

| | |
|---|---|
| α-Tocopherol | 770 mg/kg |
| β-Tocopherol | 30 mg/kg |
| γ-Tocopherol | 10 mg/kg |
| δ-Tocopherol | < 5 mg/kg |

Analyse des gereinigten Oels von Cucurbita pepo L. gemäss Aufarbeitungsbeispiel 2 :

| | |
|---|---|
| Freie Fettsäure | 0,66 |
| Brechungsindex (nD 50°C) | 1,4627 |
| Peroxidzahl | 5,3 |

Wichtigste Fettsäureverteilung in % :

| | |
|---|---|
| C 16 | 10,6 |
| C 18 | 4,9 |
| C 18 : 1 | 29,1 |
| C 18 : 2 | 54,5 |

Im Gaschromatogramm sind folgende gesättigte Fettsäuren:

| | |
|---|---|
| Behensäure | $C_{22}H_{44}O_2$ |
| Arachidsäure | $C_{20}H_{40}O_2$ |
| Stearinsäure | $C_{18}H_{36}O_2$ |
| Palmitinsäure | $C_{16}H_{32}O_2$ |
| Myristinsäure | $C_{14}H_{28}O_2$ |
| Laurinsäure | $C_{12}H_{24}O_2$ |
| Capronsäure | $C_6H_{12}O_2$ |
| Isocapronsäure | $C_6 H_{12}O_2$ |
| Valeriansäure | $C_5 H_{10}O_2$ |
| Isovaleriansäure | $C_5 H_{10}O_2$ |
| Buttersäure | $C_4 H_8 O_2$ |
| Propionsäure | $C_3 H_6 O_2$ |
| Essigsäure | $C_2 H_4 O_2$ |

und folgende ungesättigte Fettsäuren:

| | |
|---|---|
| Oleinsäure | $C_{18}H_{34}O_2$ |
| Linolsäure | $C_{18}H_{32}O_2$ |
| Linolensäure | $C_{18}H_{30}O_2$ |

nachweisbar.

Die präparative Trennung/Reinigung erfolgt auf DC-Platten 20x100 cm mit einer Kieselgelschicht Merck 60PF 254, bzw. auf DC-Fertigplatten 20x20 cm RP-18F254, 0,25 mm

Verdünnung: 1:1 mit TBME / bzw. $CH_3Cl$
Fliessmittel: Chloroform/Methanol/Wasser/Essigsäure 100% 90/10/1/0,5
Elutionsmittel: $CH_2Cl_2$ : MeOH 70/30
Auftragung auf die Nano DC Fertigplatte in einer 10%-Lösung mit Myristinsäureisopropylester

Die anschliessende Reinigungsstufe wird auf einer C-18 HPLC-Säule vorgenommen; Verdünnung 1:1 THF; Gradientenbildung. Eluents: a) $H_2O$ + 0,1 % TFA b) ACN + 0,1 % TFA

Die Spektralanalyse weist auf, dass in diesem lipophilen Anteil freie Sterole und/oder deren Fettsäureester und/oder Glykoside der Formeln vorkommen:

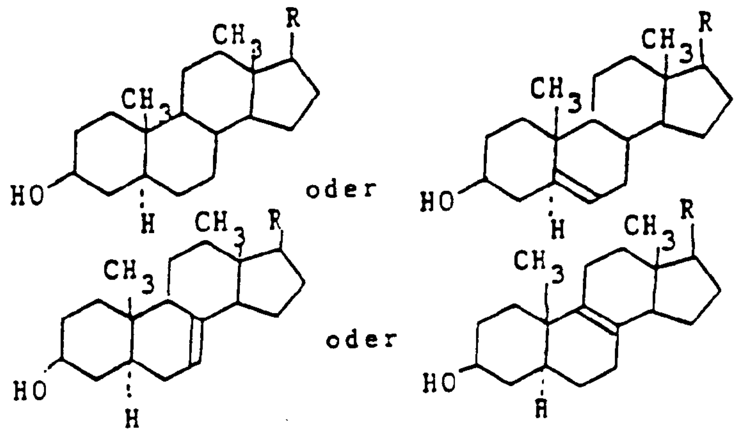

oder

oder

oder

worin R eine $C_1$ - $C_{10}$-Alkyl- oder eine $C_2$ - $C_{10}$-Alkenylgruppe darstellt. Die Alkyl-, bzw. Alkenylgruppen bei R können geradkettig oder verzweigt sein und haben in der Kette vorzugsweise 8 - 10 Kohlenstoffatome. Beispiele solcher Gruppen sind u.a.:

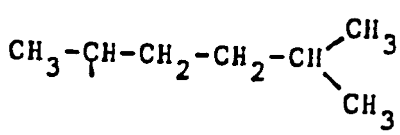

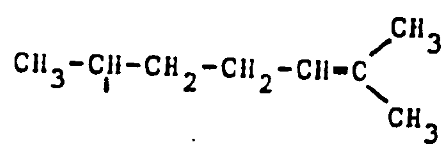

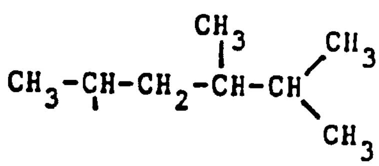

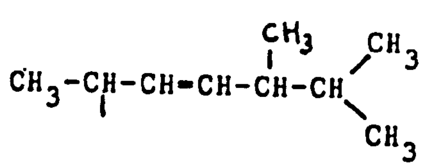

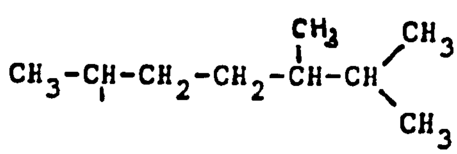

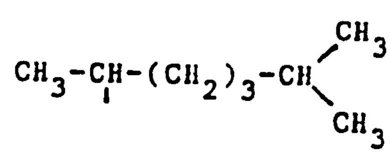

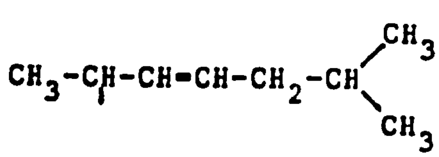

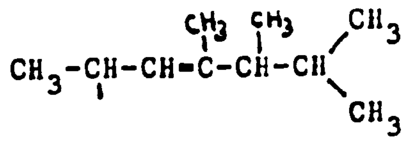

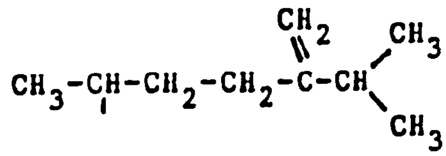

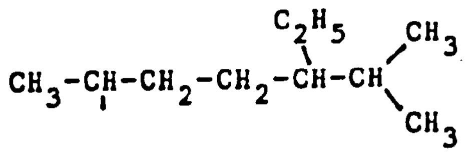

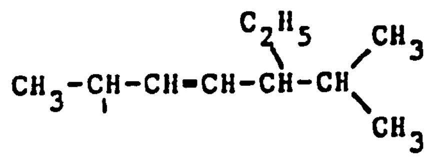

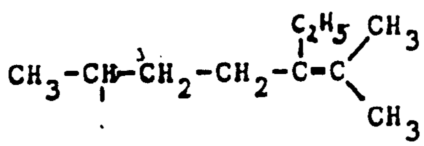

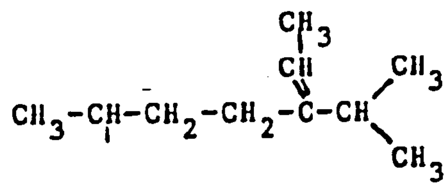

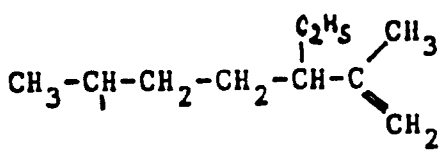

und

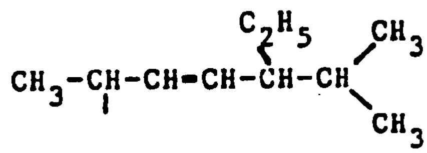

Die wichtigsten dieser Phytosterole sind:

24 β-Aethylcholesta - 5,22,25 - trien - 3β-ol (25,27 Dehydroporiferasterol)
24 β-Aethylcholesta - 5,25 (27) - dien - 3β-ol (Clerosterol)
24 z-Aethylidencholest - 5 - en - 3β-ol (Isofucosterol)
24 α-Aethylcholesta - 5,22 - dien -3β-ol (Δ5-Stigmasterol)
24 α-Aethylcholest - 5 - en - 3β-ol (Δ5-Sitosterol)
24 α-Methylcholest - 5 - en - 3β-ol (Campesterol)
24 β-Methylcholest - 5,25 (27) - dien - 3β-ol (Codisterol)
24 α-Aethylidencholest - 5 - en - 3β-ol (Δ5-Avenasterol)
24 α-Aethyl- 5α - cholesta - 7,22 - dien - 3β-ol (Spinasterol)
24 α-Aethyl - 5α - cholesta - 7 - en - 3β-ol (22-Dihydrospinasterol)
24 α-Aethylidencholest - 7 - en - 3β-ol (Δ7-Avenasterol)
24 β-Methyl - 5α - cholesta 7,25 (27) dien - 3β-ol (25,27-Dehydrofungisterol)
24 α-Aethyl - 5α - cholesta - 7,22 - dien - 3β-ol (Δ7-Stigmasterol )
24 α-Aethylcholest - 7 - en - 3β-ol (Δ7-Sitosterol)
24 α-Aethyl - 5α - cholesta - 8,22 - dien - 3β-ol
und
24 β-Aethyl - 5α - cholesta - 8,25(27)-dien-3β-ol

3. Aufarbeitungsbeispiel für die wässrige mittlere Phase gemäss dem Aufarbeitungsbeispiel 1

Das Filtergut von 16 l der wässrigen mittleren Phase gemäss dem Arbeitsbeispiel 1 wird zuerst einer Ultrafiltration mit einem 0,2 μm Pellicon Kassettenfilter (Millipore, Zürich) Nr. GVLP 00005, Filterfläche 0,47 $m^2$ unterworfen. Die Filtergeschwindigkeit beträgt 5 l/h bei 3,5 bar. Das schlammige Retentat von 0,8 l, welches die restlichen Zelltrümmer enthält, wird verworfen. Das Filtrat von 15 Litern wird nochmals ultrafiltriert mithilfe eines Pellicon Kassettenfilters mit einer Ausschlussgrenze von 30'000 Dalton [δ] (Millipore Nr. PTTK 00005, Filterfläche 0,47 $m^2$). Die Filtergeschwindigkeit beträgt 3 l/h bei 2,5 bar. Das Filtrat von 0,85 l wird gefriergetrocknet. Man erhält eine bräunliche pulverförmige Substanz mit einem Gewicht von 127 g.

Diese Substanz enthält als Hauptbestandteil 38,1 % eines Proteins mit einem Molekulargewicht von ca. 19'100 δ und einem Gehalt an folgenden Aminosäuren:

Aminosäuregehalt des Proteins von ca. 19'100 δ :

| | n Mol | M.Gewicht netto | Produkt δ |
|---|---|---|---|
| Asp. | 14,8 | 115 | 1'702 |
| Thr. | 7,7 | 101 | 778 |
| Ser. | 11,5 | 87 | 1'001 |
| Glu. | 24,4 | 103 | 2'513 |
| Gly. | 27,8 | 57 | 1'585 |
| Ala. | 10,8 | 71 | 767 |
| Cys. | 4,8 | 103 | 494 |
| Val. | 10,3 | 99 | 1'020 |
| Met. | 5,5 | 131 | 721 |
| iso-Leu. | 8,3 | 113 | 938 |
| Leu. | 12,5 | 113 | 1'413 |
| Tyr. | 2,9 | 163 | 473 |
| Phe. | 4,8 | 147 | 706 |
| His. | 3,4 | 137 | 466 |
| Lys. | 8,6 | 128 | 1'101 |
| Arg. | 14,9 | 156 | 2'324 |
| Pro. | 10,9 | 97 | 1'057 |
| | | | Σ 19'059 |

Die wässrige Phase des Extraktes aus den Samen von Cucurbita pepo L. ergibt nach dem Lyophilisieren eine hellbraune Substanz, welche 17,5 % eines Proteins von 8734 δ und 41,4 % eines Proteins von 20'702 δ enthält.

Das Protein von 8'734 δ hat folgenden Aminosäuregehalt:

| | n Mol | M.Gewicht netto | Produkt δ |
|---|---|---|---|
| Asp. | 6,6 | 115 | 759 |
| Thr. | 2,2 | 101 | 222 |
| Ser. | 5,7 | 87 | 496 |
| Glu. | 15,3 | 103 | 1'576 |
| Gly. | 21,3 | 57 | 1'214 |
| Ala. | 6,1 | 71 | 433 |
| Cys. | 1,8 | 103 | 185 |
| Val. | 4,0 | 99 | 396 |
| Met. | 2,3 | 131 | 301 |
| iso-Leu. | 3,1 | 113 | 350 |
| Leu. | 6,2 | 113 | 701 |
| Tyr. | 2,0 | 163 | 326 |
| Phe. | 2,6 | 147 | 382 |
| His. | 1,9 | 137 | 260 |
| Lys. | 5,1 | 128 | 653 |
| Arg. | 0,9 | 156 | 140 |
| Pro. | 3,5 | 97 | 340 |
| | | | Σ 8'734 |

Der Gehalt an Aminosäuren des Proteins von 20'702 δ beträgt:

| | n Mol | M.Gewicht netto | Produkt δ |
|---|---|---|---|
| Asp. | 21,3 | 115 | 2'450 |
| Thr. | 8,2 | 101 | 828 |
| Ser. | 13,0 | 87 | 1'131 |
| Glu. | 0,8 | 103 | 82 |
| Gly. | 17,0 | 57 | 969 |
| Ala. | 16,6 | 71 | 1'179 |
| Cys. | 0,9 | 103 | 93 |
| Val. | 13,2 | 99 | 1'307 |
| Met. | 5,1 | 131 | 668 |
| iso-Leu. | 9,3 | 113 | 1'051 |
| Leu. | 17,8 | 113 | 2'011 |
| Tyr. | 6,8 | 163 | 1'108 |
| Phe. | 11,6 | 147 | 1'705 |
| His. | 5,0 | 137 | 685 |
| Lys. | 7,4 | 128 | 947 |
| Arg. | 22,3 | 156 | 3'479 |
| Pro. | 10,4 | 97 | 1'009 |
| | | | Σ 20'702 |

Ein Teil des Filtrates nach der Ultrafiltration mit dem 0,2 µm Pellicon Kassettenfilter wird im Vakuum eingeengt und anschliessend lyophilisiert. Eine Flachgel-Elektrophorese zeigt auf, dass dieses Lyophilisat überdies noch Proteine mit 94'000 δ, 67'000 δ, 43'000 δ und 30'000 δ enthält.

In vergleichbarer Weise wurde der Aminosäuregehalt von zwei hauptsächlichen Proteinen mit einer Molekularmasse von 19'051 δ, bzw. 30'451 δ festgestellt, welche aus dem wässrigen Extrakt von Cucurbita maxima Samen gemäss Aufarbeitungsbeispiel 1 gewonnen wurden.

5. Aufarbeitungsbeispiel: Herstellung von biopharmazeutischen Arzneimitteln in Form von Kapseln, Tabletten, Suppositorien oder Ampullen.

Die Aufarbeitung der vorstehend beschriebenen spontan dispergierbaren Konzentrate zu therapeutischen Systemen wird nach folgender Grundrezeptur vorgenommen:

| | EURISOL® 500 mg Kapseln 20 G Einh. | | EURIKIN® 250 mg Kapseln 17,5 G Einh. | |
|---|---|---|---|---|
| | pro Dosis mg | Ansatz g | pro Dosis mg | Ansatz g |
| Reinextrakt aus Helianthus annuus | 15 | 3 | | |
| Reinextrakt aus Cucurbita maxima | | | 15 | 6 |
| Oelanteil Hel.a. | 225 | 45 | | |
| Cuc.m. | | | 105 | 42 |
| ß-Carotin | 10 | 2 | 5 | 2 |
| α-Tocopherol | 5 | 1 | 2,5 | 1 |
| Maiskeimoel Maydis Germinis Oleum | 200 | 40 | 100 | 40 |
| Isopropylmyristat Myristinsäure-Isopropylester (Fluka) | 10 | 2 | 5 | 2 |
| Kieselgel 60 reinst | 25 | 5 | 12,5 | 5 |
| Invadin® JFC 800 % | 5 | 1 | 2,5 | 1 |
| Mischemulgator 3873 | 5 | 1 | 2,5 | 1 |
| Σ | 500 | 100 | 250 | 100 |

Für die Anwendung in Form einer Brausetablette ist wegleitend

| | | EURISOL• | EURIKIN• |
|---|---|---|---|
| | | BRAUSETABLETTEN | |
| | | pro Dosis mg | pro Dosis mg |
| Reinextrakt | Hel.ann. | 9 | |
| | Cuc.max. | | 9 |
| Oelanteil | Hel.ann. | 20 | |
| | Cuc.max. | | 20 |
| ß-Carotin | 200 ppm. | 1 | 1 |
| Proteinanteil | Hel.ann. | 30 | |
| | Cuc.max. | | 30 |
| Emulgatoren | | 10 | 10 |
| Hanfzellulose | | 355 | 355 |
| $NaHCO_3$ | | 975 | 975 |
| Zitronensäure crist. | | 1000 | 1000 |
| evtl. Geschmacksstoffe | | - | - |
| | | Σ 2400 mg | 2400 mg |

N.B.: EURISOL• und EURIKIN• sind eingetragene Marken (registered trademarks ™) der Firma MARIGEN S.A., RIEHEN

Biologische Prüfungen.

Die antitumorale Wirkung der Wirkstoffe, sowie der spontan emulgierbaren Konzentrate gemäss den Aufarbeitungsbeispielen No. 1 - 4 wird anhand folgender Prüfungsergebnisse bestätigt:

1. in vitro-Tests mit geeigneten Tumorzell-Linien

Es wurde ein biologisches Assay-System entwickelt, das mit Mikrotiterplatten und Verdünnungsreihen arbeitet. Angesetzt werden je $10^4$/ml Tumorzellen in Kulturmedium RPMI 1640 mit 10 % fötalem Kalbserum inaktiviert (GIBCO); sie werden so undicht ausgesät, dass sie während des Assays wachsen können, in sog. nichtkonfluenten Monolayers. Die Probenzugabe erfolgt nach 6 - 24 Stunden, mit 100 µl pro Reihe, die man im 1. Loch mit 100 µl Medium versetzt. Davon wird die Hälfte entnommen und in das folgende Loch eingebracht, wieder mit 100 µl Medium versetzt, usf. Es entsteht eine geometrische Verdünnungsreihe $n^{1/2}$.

Die Proben werden im Plaque Assay während 3-5 Tagen bei 37° C mit 3½ % $CO_2$ inkubiert. Anschliessend färben/ fixieren mit 0,1 % Kristallviolett (Fluka, Buchs) in einer Lösung von 70 % Methanol, 1 % Formaldehyd, 29 % Wasser. Die Auswertung wird am Mikroskop vorgenommen, Vergrösserung 300-fach. Man bestimmt die grösste cytotoxische Verdünnung. Die quantitative Auswertung lässt sich auch mittels Scanning und Absorptionsmessung am Spektrophotometer vornehmen.

## AUSWERTUNG DER ERGEBNISSE

| Tumorlinie<br>Präparat | TSA<br>In Verdünnung wirksam bis 1 : | BNY | 2002 | NMC |
|---|---|---|---|---|
| ***Helianthus annuus*** | | | | |
| Reinextrakt 1:10 | 327'600 | 163'840 | | |
| 1:100 | | ≥12'800 | 6'400 | ≥12'800 |
| Oelanteil 1:10 | >4096 | 40'960 | | 5'120 |
| Konzentrat 1:10 | ≥1'280 | ≥1'280<br>>>2'560 | - | |
| ***Cucurbita maxima*** | | | | |
| Oelanteil 1:10 | | | | ≥1'280 |
| Konzentrat 1:10 | >1'280 | >>2'560 | | |

T S A : murines Adenocarcinom (spontanes Mamacarcinom)
Prof. Guido Forni, Istituto di Microbiologia
Università di Torino, Scuola di Medicina
BNY : humanes Melanom (Biotechnologie CIBA-GEIGY)
2002 : FLOW humane Linie (Embryonen-Lungenfibroblast)
NMC : Neuroblastoma human

### 2. in vivo-Tests an der Maus

Die Prüfungen in vivo wurden an der weiblichen Maus vorgenommen. Man verwendete alte Zuchttiere Balb-c mit einem Körpergewicht von 28 - 32 g (Charles River, Milano). Als durchgehender Vergleichsmassstab diente das spontane Adenocarcinom TSA, eine murine Tumor-Zelllinie, welche uns von Prof. Guido Forni, Istituto di Microbiologia, Universita di Torino, Scuola di Medicina, regelmässig zur Verfügung gestellt wurde.

### Fütterungsversuch

Durchgeführt von Prof. Giorgio Rivara, Coordinatore sanitario USL ed Ospedale Maggiore San Giovanni Battista, LE MOLINETTE, Torino. Die Verabreichung von "Pellets", hergestellt aus vorgekeimtem, lyophilisiertem Homogenat aus den Samen, bzw. Samenkernen von Helianthus annuus einerseits und Cucurbita maxima/pepo anderseits und täglich als Alleinfutter eingesetzt, wurde gut aufgenommen und gut vertragen. Es hat während über 60 Tagen zu keinerlei Mangelerscheinung oder zu sichtbaren Nebenwirkungen geführt. Die Homogenate können in dieser Form als untoxisch bezeichnet werden.

### Gavage

Verschiedene Gruppen von je 5 Versuchtstieren wurden mit normalem Versuchsfutter (NAFAG-Alleinfutter No. 850 der Nährund Futtermittel AG, Gossau) gefüttert.

Die Kontrollgruppe erhält einmalig eine Injektion inguinal (in die linke Flanke) von 80 - 100'000 Zelleinheiten der tumoralen Linie TSA, die sehr gut fasst und subkutan regelmässig wächst und eine feste Tumormasse ausbildet. Im Abstand von 14 Tagen nach der Setzung (durchschnittliche Latenzzeit), wird das entstandene Neugewebe palpiert; ferner nach 21 und nach 28, evtl. 35 Tagen. Man bestimmt das Mittel aus Länge und Breite der festen, abgegrenzten Tumormasse unter der Haut. (Nachkontrolle mittels Sektion).

Für die Prüfung der Wirksamkeit der Präparate am lebendigen Organismus wird den übrigen Versuchstieren zusätzlich zum normalen Versuchsfutter täglich 0,2 ml eines Versuchspräparates mittels einer Sonde eingegeben. Nach 3 - 4 Tagen der Angewöhnung wird der Tumor gesetzt, ebenfalls inguinal mit 80 bis 100'000 TSA-Einheiten, wie bei den Kontrollen. Er fasst ausnahmlos - in der Gruppe und bei Wiederholungen - und liefert sehr gut vergleichbare Ergebnisse, was erlaubt, die statistischen Gruppen verhältnismässig klein zu halten.

Injektion s.c.

Die Versuchsanlage ist gleich wie bei der Gavage. Statt zu sondieren werden die zu prüfenden Präparate durch Injektion inguinal (s.c. rechte Flanke) mit 0,2 ml 3 mal pro Woche appliziert.

**AUSWERTUNG DER ERGEBNISSE**

| Präparat TSA nach | TUMORMASSE Σ½ mm 14 | 21 | 28 | 35 Tagen |
|---|---|---|---|---|
| Kontroll-gruppe | 6 | 8 | 13 | 17 |
| Cucurbita max. Vollkonzentrat Pellets per os | 0 | 0 | 0 | 0 |
| Helianthus an. Vollkonzentrat Pellets per os | 0<br>LK<br>0<br>0 | 0<br>LK<br>+<br>++ | 0<br>0<br>2<br>3 | 0<br>0<br>2<br>5 |
| Helianthus an. Sp.d. Konzentrat 0,2 ml 3xWo. s.c. | 0<br>0<br>0<br>2 | 0<br>0<br>++<br>4 | 0<br>0<br>3<br>6 | 0<br>2<br>6<br>8 |
| Helianthus an. Grasso + NAFAG per os | 0<br>0<br>1<br>2 | 0<br>0<br>2<br>4 | 0<br>0<br>6<br>6 | 0<br>0<br>6<br>8 |
| Helianthus an. Sp.d. Konzentrat 0,2 ml/d per os | 0<br>0<br>2<br>2 | 0<br>0<br>4<br>5 | | |

L.K. = Lymphknoten + und ++ Schwellung

Verfahrensbeispiele zur halbsynthetischen Herstellung von erfindungsgemäss zu verwendenden Fettsäureestern von Sterolen:

1) Zu 1 g trans-2-Dodecensäure (MW 198,31) in 50 ml Toluol werden 1,5 g Thionylchlorid (MW 118,77;

Ueberschuss) und 10 Tropfen Dimethylformamid zugesetzt. Nach 20 Stunden bei 4° C wird dem Reaktionsgemisch 1 g β-Sitosterol (MW 414,72) zugefügt. Nach nochmaligem Stehen bei 22 °C während 20 Stunden wird das Lösungsmittel im Vakuum abdestilliert und der oelige Rückstand auf einer Kieselgelsäule chromatographiert; Eluiermittel Hexan/Essigester 9 : 1. Man gewinnt aus der ersten Fraktion das β-Sitosterol-Dodecenoat (das C 12:1-Sitosterol) als gelbes Oel mit einem Brechungsindex $n_D$ 20 °C = 1,5118

Auf analoge Weise werden auch folgende Verbindungen hergestellt:

| | |
|---|---|
| CHOLESTERYL-DODECENOAT | (C 12:1-CHOLESTEROL) |
| CHOLESTERYL-LINOLEAT | (C 18:2-CHOLESTEROL) |
| CHOLESTERYL-LINOLENAT | (C 18:3-CHOLESTEROL) |
| β-SITOSTEROL-LINOLEAT | (C 18:2-Sitosterol) |
| β-SITOSTEROL-LINOLENAT | (C 18:3-Sitosterol) |
| STIGMASTEROL-DODECENOAT | (C 12:1-Stigmasterol) |
| STIGMASTEROL-LINOLEAT | (C 18:2-Stigmasterol) |
| STIGMASTEROL-LINOLENAT | (C 18:3-Stigmasterol) |

2) 3 g Undecenoylchlorid und 2 g β-Sitostcrol werden in 150 ml Tetrahydrofuran während zwei Stunden am Rückfluss bei 80°C gehalten. Nach dem Zusatz von 2 ml Pyridin oder Dimethylformamid wird die Reaktionslösung nochmals eine Stunde am Rückfluss auf 80°C erwärmt.

Das Lösungsmittel wird am Rotavapor abdestilliert und der Rückstand in Acetonitril umkristallisiert. Man erhält das β-Sitostcrol-Undecenoat (C 11:1-Sitosterol), mit einem Schmelzpunkt von 68,3 °C.

Auf analoge Weise werden auch folgende Fettsäureester hergestellt:

| | | |
|---|---|---|
| CHOLESTERYL-CAPRYLAT | (C 8:0-CHOLESTEROL) | Smp.: 106,6°C |
| CHOLESTERYL-UNDECENOAT | (C 11:1-CHOLESTEROL) | Smp.: 78,9°C |
| CHOLESTERYL-LAUROYLAT | (C 12:0-CHOLESTEROL) | Smp.: 79,4°C |
| CHOLESTERYL-PALMITAT | (C 16:0-CHOLESTEROL) | Smp.: 77-79°C |
| CHOLESTERYL-OLEAT | (C 18:1-CHOLESTEROL) | Smp.: 46 °C |
| β-SITOSTEROL-CAPRYLAT | (C 8:0-SITOSTEROL) | Smp.: 71,3°C |
| β-SITOSTEROL-LAUROYLAT | (C 12:0-SITOSTEROL) | Smp.: 83,3°C |
| β-SITOSTEROL-PALMITAT | (C 16:0-SITOSTEROL) | Smp.: 89,6°C |
| β-SITOSTEROL-OLEAT | (C 18:1-SITOSTEROL) | Smp.: 52 °C |
| STIGMASTEROL-CAPRYLAT | (C 8:0-STIGMASTEROL) | Smp.: 89,9°C |
| STIGMASTEROL-UNDECENOAT | (C 11:1-STIGMASTEROL) | Smp.: 85,7°C |
| STIGMASTEROL-LAUROYLAT | (C 12:0-STIGMASTEROL) | Smp.: 97,1°C |
| STIGMASTEROL-PALMITAT | (C 16:0-STIGMASTEROL) | Smp.: 91 °C |

3) Zu 2 g Dodecenylsäure in 200 ml Chloroform werden 1,8 g 1,1'-Carbonyl-diimidazol zugegeben. Man erwärmt die Re-aktionslösung während 12 Stunden auf 30°C und setzt dann 4,127 g Stigmasterol zu. Nach weiteren 12 Stunden bei 30°C wird das Lösungsmittel abdestilliert und der Rückstand in 150 ml Essigester aufgenommen. Diese Lösung schüttelt man einmal mit 1/10 N Salzsäure und einmal mit 1/10 N Natronlauge aus. Nachdem der Essigester abdestilliert worden ist, nimmt man den Rückstand in wenig Hexan/Essigester (9 : 1) auf. Diese Lösung wird sodann auf einer Silicagelsäule mit Hexan/Essigester (9:1) chromatographiert. Man erhält das reine Stigmasterol-Dodecenoat (C 12:1-Stigmasterol) mit einem Brechungsindex

von $n_D$ 20 °C = 1,4913

Auf analoge Weise werden auch folgende Verbindungen hergestellt:

| | |
|---|---|
| β-SITOSTEROL-DODECENOAT | (C 12:1-SITOSTEROL) |
| CHOLESTERYL-DODECENOAT | (C 12:1-CHOLESTEROL) |
| β-SITOSTEROL-OLEAT | (C 18:1-SITOSTEROL) |
| STIGMASTEROL-OLEAT | (C 18:1-STIGMASTEROL) |

Vgl. im übrigen die in der technischen Beilage gegebenen Messdaten: 1/6 bis 4/6.

<u>Verfahrensbeispiel zur halbsynthetischen Herstellung von erfindungsgemäss zu verwendenden Glucosiden von Sterolen: Stigmasterol-β-d-Glucosid</u>

In einen 100 ml Dreihalskolben mit Destillieraufsatz, Tropftrichter und Magnetrührer gibt man 0,5 g Stigmasterol, 0,6 g Silbercarbonat und 20 ml Benzol. In den Tropftrichter wird eine Lösung von 1,35 g Acetobromglucose in 30 ml Benzol gebracht.

Mit einem Oelbad wird die Benzollösung im Kolben erwärmt, bis das Benzol schwach destilliert. Unter Rühren wird nun die Benzollösung aus dem Tropftrichter innerhalb von ca. 45 min. zugetropft. Die Reaktionsmischung wird noch 60 min. knapp unter dem Siedepunkt gerührt, etwas abgekühlt, filtriert und im Vakuum zur Trockene eingedampft. Der Rückstand wird in 20 ml heissem Alkohol aufgelöst, mit 5 ml Wasser versetzt und über Nacht bei 0 - 5 °C stehen gelassen.

Die ausgefallenen Kristalle werden abfiltriert, in 20 ml Methanol warm aufgelöst und bei Zimmertemperatur mit 5 %-iger Lösung von Natriummethylat in Methanol auf pH ca. 12,5 gestellt und 30 min. bei diesem pH gelassen. Anschliessend wird mit Essigsäure auf pH ca. 7 gestellt und das Gemisch zur Trockene genommen.

Der Rückstand wird in 20 ml Chloroform-Methanol 1:1 aufgenommen, 1 g Kieselgel zugeführt und das Ganze im Vakuum wieder zur Trockene gebracht.

Der Rückstand wird in 30 ml Chloroform-Methanol 99:1, gesättigt mit Wasser, aufgeschlämmt. Die Suspension wird auf eine Chromatographiesäule aus 20 g Kieselgel im gleichen Lösungsmittelsystem geschlämmt. Die Säule wird mit je 100 ml des gleichen Systems, Chloroform-Methanol 9:1 und 8:1 (immer wassergesättigt) eluiert. Das Produkt erscheint mit ChloroformMethanol 8:2 (wassergesättigt). Die Lösung wird im Vakuum zur Trockene gebracht, in 10 ml heissem Alkohol aufgeschlämmt, mit 3 ml Wasser versetzt und über Nacht bei 0 - 5 °C stehen gelassen. Anschliessend wird das Produkt abfiltriert und im Vakuum bei I.T. 60 °C während 8 Std. getrocknet. Man erhält ca. 30 mg kristallines Produkt.

Rf-Wert im Laufmittel Chloroform-Methanol-Wasser 15:5:2 (untere Phase): 0,4. Schmelzpunkt: 255,9 - 256,7 °C.

In gleicher Weise lassen sich Cholesterol, Sitosterol, Oestradiol und andere Steroide zu Glucosiden verarbeiten. Vgl. auch die in der technischen Beilage 5/6 und 6/6 gegebenen Daten.

<u>Zusammensetzungsbeispiele von erfindungsgemässen spontan dispergierbaren Mitteln, welche als Wirkstoffe Fettsäureester von Sterolen gemäss den Formeln I bis X enthalten.</u>

a) 7,5 Gew.% eines Fettsäureesters von Sterolen (Formeln I bis X)
   7,5 Gew.% Isopropylmyristat (= Myristinsäure-Isopropylester)
   42,5 Gew.% Mischemulgator Diphasol® 3873 (CIBA-GEIGY)
   42,5 Gew.% Invadin® JFC 800% (CIBA-GEIGY)

Diphasol® 3873 ist ein Mischemulgator, bestehend aus je 50% der beiden Verbindungen mit Formel

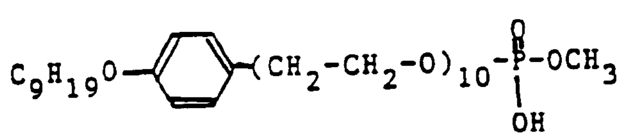

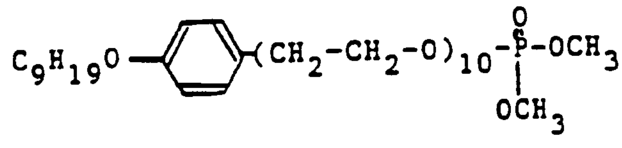

Invadin® JFC 800% (CIBA-GEIGY) ist ein tert. Octylphenylpolyoxyäthylenäther mit 9 - 10 Oxyäthylengruppen

b) 7,5 - 15 Gew.% eines oder mehrerer Fettsäureester von Sterolen der Formeln I bis X,

0 - 40 Gew.% Isopropylmyristat, Olivenoel oder Sonnenblumenoel kalt gepresst und unbehandelt; Maiskeimoel, Weizenkeimoel oder Distelöl.

22,5 - 42,5 Gew.% Diphasol® 3873 und

22,5 - 42,5 Gew.% Invadin® JFC 800 %

c) 7,5 - 15 Gew.% eines oder mehrerer Fettsäureester von Sterolen der Formeln I bis X,

0 - 40 Gew.% Isopropylmyristat

22,5 - 42,5 Gew.% Invadin® JFC 800%

22,5 - 42,5 Gew.% Soprophor® FL, bzw. FLK (Rhône-Poulenc)

Aufarbeitungsbeispiel: Herstellung von biopharmazeutischen Arzneimitteln in Form von Micropellets, Kapseln, Tabletten, Suppositorien oder Ampullen, welche als Wirkstoffe Fettsäureester von Sterolen gemäss den Formeln I bis X enthalten

Die Aufarbeitung der vorstehend beschriebenen spontan dispergierbaren Konzentrate zu therapeutischen Systemen wird nach folgender Grundrezeptur vorgenommen:

| | 250 mg Kapseln | 500 mg Kapseln |
|---|---|---|
| Fettsäureester von Sterolen: **MARIGENOL•-Konzentrat** (evtl. gelöst in Olivenoel extra vergine, bzw. Sonnenblumenöl, Weizenkeimöl, Distelöl oder Maiskeimöl, kaltgepresst, unbehandelt) | 1-25 mg | 2-50 mg |
| Dest. Wasser (wird lyophilisert) | (250 mg) | (500 mg) |
| Lactose | 160-184 mg | 325-373 mg |
| Kristallzellulose | 40 mg | 75 mg |
| Syloid 244 H | 25 mg | 50 mg |
| | 250 mg | 500 mg |

Für die Herstellung von sog. **"multiple units"-Arzneimitteln** ist wegleitend:

| KONZENTRATE | pro Dosis | pro Ansatz |
|---|---|---|
| Konzentrat MARIGENOL® mit einem oder mehreren Fettsäureester-Sterolen und/oder Sterol-Glucosiden | 20 mg | 20 g |
| Isopropylmyristat | 120 mg | 120 g |
| Invadin® JFC 800 % | 240 mg | 240 g |
| Soprophor® FLK | 120 mg | 120 g |
| Sorbit-Lösung | 15 mg | 15 g |
| Sulfo-Salicylsäure | 15 mg | 15 g |
| Vitamin A | 10 mg | 10 g |
| α-Tocopherol | 5 mg | 5 g |
| Polyethylenglycol 6000 | 10 mg | 10 g |
| Syloid® 244 H | 10 mg | 10 g |
| MARIGENOL®-Konzentrat | 565 mg | 565 g |

| M I C R O P E L L E T S | | | |
|---|---|---|---|
| Lactose/Zellulose-Kern 80:20 | | 495 g | 28 % |
| MARIGENOL®-KONZENTRAT | | 565 g | 32 % |
| Schutzlack hydrophob (z.B. mit Hydroxypropylmethyl-Cellulose oder EA-MMA-HEMA-Copolymer oder EUDRAGIT® E) | | 70 g | 4 % |
| EUDRAGIT® L 100 /L 30 D | T.S. | 635 g | 36 % |
| | | 1765 g | |

Herstellung in der Wirbelschicht, bzw. dem Rotationsbett.

N.B. MARIGENOL® ist eine eingetragene Marke (reg.™) der Firma MARIGEN S.A., RIEHEN.

BIOLOGISCHE PRUEFUNGEN

Die antitumorale Wirkung der Fettsäureester von Sterolen wird anhand folgender Prüfungsergebnisse bestätigt:

1. in vitro-Tests mit geeigneten Tumorzell-Linien

Es wurde ein biologisches Assay-System entwickelt, das mit Mikrotiterplatten und Verdünnungsreihen ar-

beitet. Angesetzt werden je $10^4$/ml Tumorzellen in Kulturmedium RPMI 1640 mit 10% fötalem Kalbserum inaktiviert (GIBCO); die Zellen werden so undicht ausgesät, dass sie während des Assays wachsen können, in sog. nichtkonfluenten Monolayers. Die Probenzugabe erfolgt nach 6-24 Stunden, mit 100 µl pro Reihe, die man im 1. Loch mit 100 µl Medium versetzt. Daraus wird die Hälfte entnommen und in das folgende Loch eingebracht, wieder mit 100 µl Medium versetzt, usf. Es entsteht eine geometrische Verdünnungreihe n½.

Die Proben werden im Plaque Assay während 3-5 Tagen bei 37°C mit 3½% $CO_2$ inkubiert. Anschliessend färben/fixieren mit 0,1% Kristallviolett (Fluka, Buchs) in einer Lösung von 70% Methanol, 1% Formaldehyd, 29% Wasser. Die Auswertung wird am Mikroskop vorgenommen, Vergrösserung 300-fach. Man bestimmt die grösste cytotoxische Verdünnung. Die quantitative Auswertung lässt sich auch mittels Scanning und Absorptionsmessung am Spektrophotometer vornehmen.

AUSWERTUNG der Ergebnisse

| MARIGENOL® Microemulsion 0,1%ig mit folgenden Fettsäureestern | Zell-Linie T S A In Verdünnung wirksam bis 1 : | | | |
|---|---|---|---|---|
| C 11:1-CHOLESTEROL | 5 h | 1'280 | 5 Tg | >640'000 |
| C 11:1-SITOSTEROL | 5 h | 40 | 5 Tg | >640'000 |
| C 12:1-SITOSTEROL | 5 h | 640 | 5 Tg | 320'000 |
| C 16:0-STIGMASTEROL | 5 h | 80 | 5 Tg | 40'000 |
| C 18:3-SITOSTEROL | 5 h | 1'280 | 5 Tg | 80'000 |
| C 12:1-SITO/18:3 TSA | 5 h | 2'560 | 4 Tg | 40'000 |
| Human Leucocytes | 1 : 1'000 | | | |
| | 24 h | alle Zellen o.k. | | |
| | 96 h | alle Zellen o.k. | | |
| | 120 h | alle Zellen o.k. | | |
| C 18:2-SITO/18:2 TSA | 5 h | 2'560 | 5 Tg | 20'000 |
| Human Leucocytes | 1 : 1'000 | | | |
| | 24 h | alle Zellen o.k. | | |
| | 96 h | alle Zellen o.k. | | |
| | 120 h | alle Zellen o.k. | | |

| | | | |
|---|---|---|---|
| LINFOMA a cellule convolute (ST4) | | | |
| C 12:1-SITO/18:3 | 0,1 % Microem. | 72 h | 1:2'500 |
| C 18:2-SITO/18:2 | 0,1 % Microem. | 72 h | 1:2'500 |
| LEUCEMIA a cellule T (PF 382) | | | |
| C 12:1-SITO/18:3 | 0,1 % Microem. | 72 h | 1:2'500 |
| C 18:2-SITO/18:2 | 0,1 % Microem. | 72 h | 1:2'500 |

T S A : murines Adenocarcinom (spontanes Mamacarcinom)
Prof. Guido Forni, Istituto di Microbiologia
Università degli Studi di Torino, Scuola di Medicina

2. in vivo-Tests an der Maus

Die Prüfungen in vivo wurden an der weiblichen Maus vorgenommen. Man verwendete alte Zuchttiere Balb-c mit einem Körper- gewicht von 28 - 32 g (Charles River, Milano). Als durchgehender Vergleichs-Massstab diente das spontane Adenocarcinom TSA, eine murine Tumor-Zelllinie, welche uns von Prof. Guido Forni, Istituto di Microbiologia, Universita degli Studi di Torino, Scuola di Medicina, regelmässig zur Verfügung gestellt wurde.

Fütterungsversuch

Zur Ermittlung der Verträglichkeit wurden täglich während 30 Tagen Dosen von 0,25 ml Microemulsion verschiedener Konzentration mit der Sonde verabreicht. Die Verträglichkeit war gut bis zu 10 mg/kg Körpergewicht.

Die LD 50 liegt bei 100 mg/kg Körpergewicht.

Gavage

Verschiedene Gruppen von je 5 Versuchstieren wurden mit normalem Versuchsfutter (NAFAG-Alleinfutter No. 850 der Nährund Futtermittel A.G., Gossau) gefüttert.

Die Kontrollgruppe erhält einmalig eine Injektion inguinal (in die linke Flanke) von 80 - 100'000 Zelleinheiten der tumoralen Linie TSA, die sehr gut fasst und subkutan regelmässig wächst und eine feste Tumormasse ausbildet. Im Abstand von 14 Tagen nach der Setzung (durchschnittliche Latenzzeit), wird das entstandene Neugewebe palpiert: ferner nach 21 und nach 28 Tagen, evtl. auch nach 35 Tagen. Man bestimmt das Mittel aus Länge und Breite der festen, abgegrenzten Tumormasse unter der Haut. (Nachkontrolle mittels Sektion).

Für die Prüfung der Wirksamkeit der Präparate am lebendigen Organismus wird den übrigen Versuchstieren zusätzlich zum normalen Futter täglich 0,25 ml eines Versuchspräparates mittels einer Sonde eingegeben. Nach 5 - 7 Tagen der Angewöhnung wird der Tumor gesetzt, ebenfalls inguinal, mit 80 - 100'000 TSA-Einheiten, wie bei den Kontrollen. Er fasst ausnahmslos - in der Gruppe und bei Wiederholungen - , was erlaubt, die statistischen Gruppen verhältnismässig klein zu halten.

Injektion s.c.

Die Versuchsanlage ist gleich wie der Gavage. Statt zu sondieren, werden die zu prüfenden Präparate durch Injektion inguinal (s.c. rechte Flanke) mit 0,25 ml 3 mal/Woche appliziert.

AUSWERTUNG der Ergebnisse

| Präparat TSA nach | Tumormasse Σ ½ L+B mm 14 | 21 | 28 Tagen |
|---|---|---|---|
| Kontrollgruppe | 6 | 8 | 13 |
| MARIGENOL® 0,1% Microemulsionen mit | | | |
| β-Sitosterol-dodecenoat | 1 | 0 | 2 |
| Stigmasterol-dodecenoat | 0 | 0 | 2 |
| β-Sitosterol-linoleat | 0 | + | 1 |
| β-Sitosterol-linolenat | + | + | 1 |

+ Schwellung

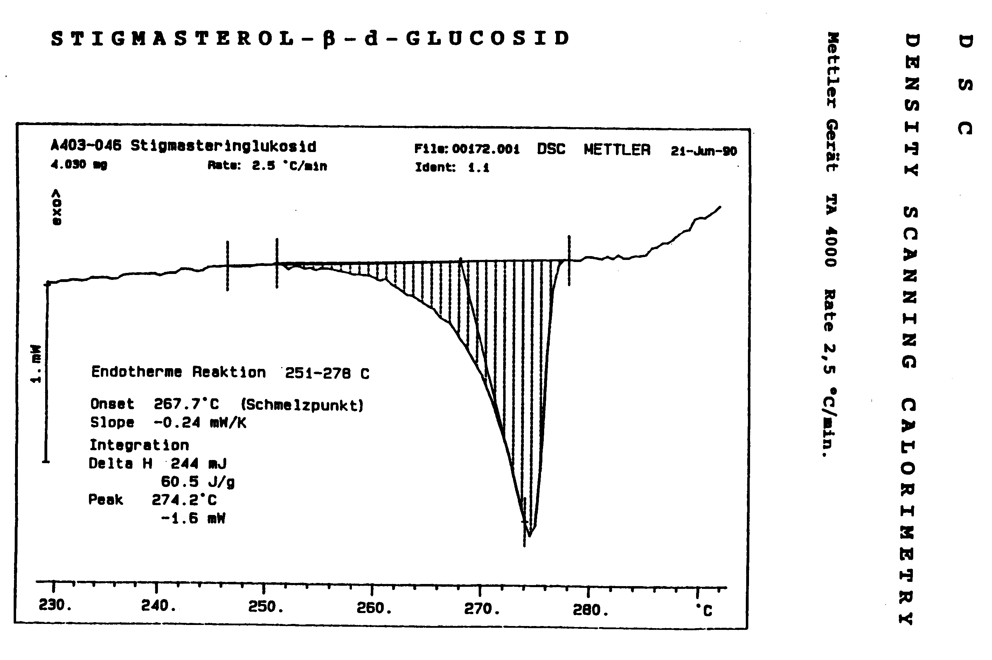

## Patentansprüche

1. Ein Verfahren zur Gewinnung der interzellulären Wirkstoffe aus den Samen von Pflanzen, welche den Familien der Korbblütler (Compositae) und der Kürbisgewächse (Cucurbitaceae) angehören, dadurch gekennzeichnet, dass man die Samen 2-4 Tage vorkeimt und dann mit destilliertem Wasser versetzt, welches 0,1 bis 3 % Mannit und 0,1 bis 4 % eines pharmaverträglichen nicht-ionogenen Tensides, bzw. Tensidgemisches - bezogen auf das Gewicht der vorgekeimten Samen - enthält; das so gewonnene Gemisch in einer Zahnkolloidmühle homogenisiert und anschliessend zentrifugiert; die entstehenden drei Phasen voneinander trennt; die untere Phase mit den Schalenteilen und den Zelltrümmern verwirft; die obere lipophile Phase nach einem Extraktionsschritt präparativ chromatographlsch reinlgt, und die mittlere wässrige Phase zunächst ultrafiltriert, daran anschliessend im Vakuum einengt und als Konzentrat lyophilisiert.

2. Ein spontan dispergierbares Konzentrat, welches als antitumorale Komponente 0,001 bis 15 Gewichts-% der gemäss Anspruch 1 hergestellten und gereinigten lipophilen Phase und/oder 0.001 bis 30 Gewichts-% des gemäss Anspruch 1 hergestellten lyophilisierten Extraktes aus den vorgekeimten Samen von Helianthus annuus L., Cucurbita pepo L. oder Cucurbita maxima Duch., sowie 0.001 bis 85 Gewichts-% eines pharmaverträglichen Tensides oder Tensidgemisches, 0.001 bis 40 Gewichts-% eines als Hydrotrop, bzw. Co-Emulgator dienenden, pharmaverträglichen Lösungsmittels oder Lösungsmittelgemisches, sowie übliche Trägerstoffe und/oder Verdünnungsmittel oder Stabilisatoren enthält.

3. Ein spontan dispergierbares Konzentrat, dadurch gekennzeichnet, dass es als antitumorale Komponente 0.001 - 15 Gewichts-% eines SterolFettsäureesters, bzw. einer Kombination solcher Ester mit Formeln

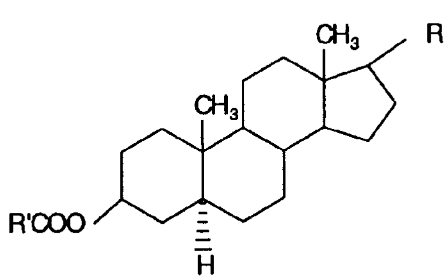

(I)

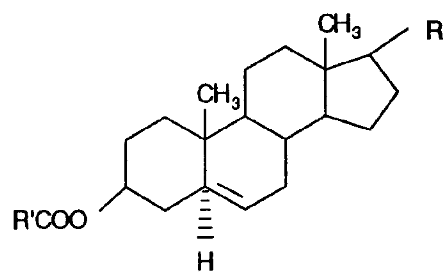

(II)

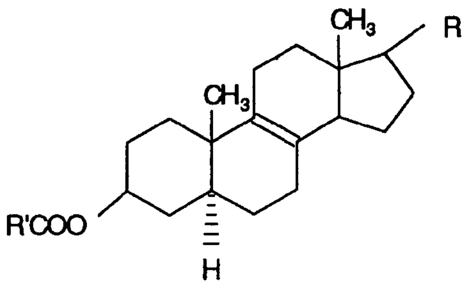

(III)

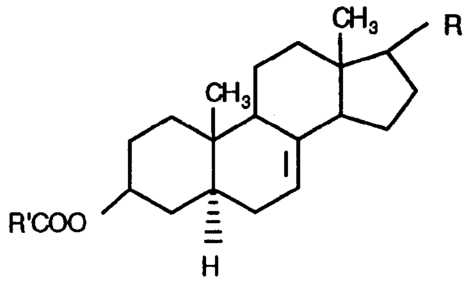

(IV)

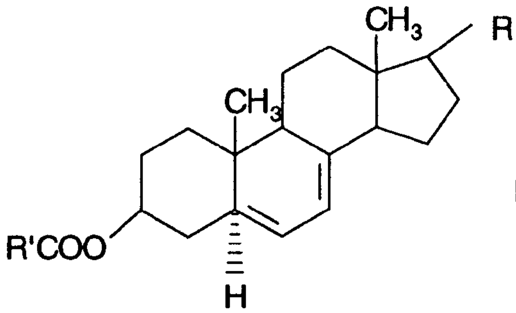

(V)

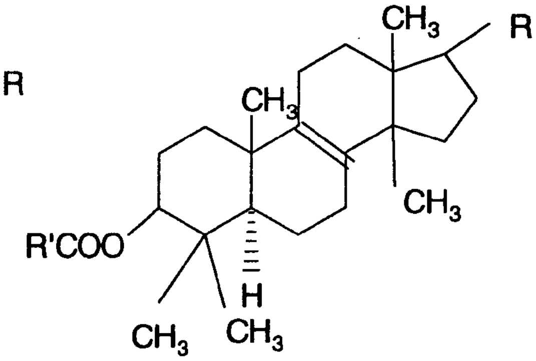

(VI)

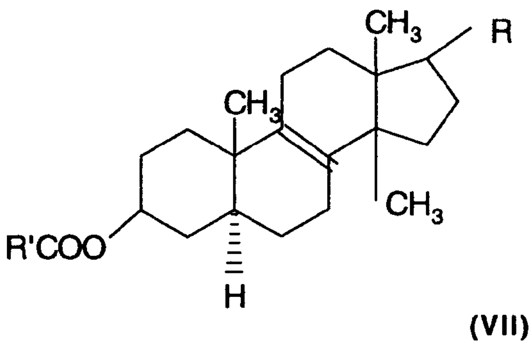

(VII)

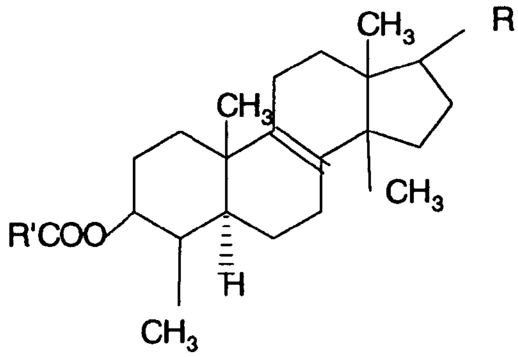

(VIII)

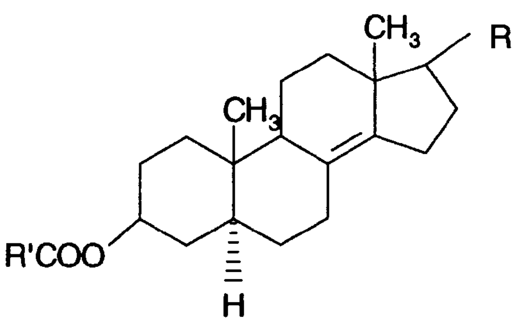

(IX)

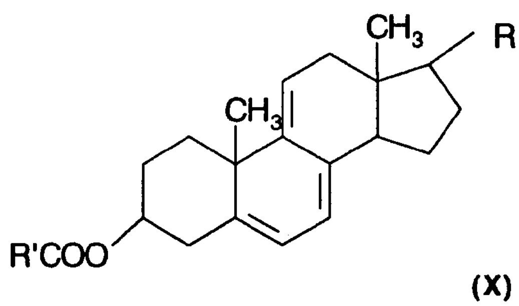

(X)

worin R eine $C_1$- bis $C_{10}$-Alkylgruppe oder eine $C_2$- bis $C_{10}$-Alkenylgruppe und R' eine $C_1$- bis $C_{32}$-Alkyl- oder eine $C_2$- bis $C_{32}$-Alkenyl-, bzw. Alkapolyengruppe bedeuten, sowie

0 bis 40 Gewichts-% eines als Hydrotrop, bzw. Co-Emulgator dienenden, pharmaverträglichen Lösungsmittels oder Lösungsmittelgemisches

0.001 bis 85 Gewichts-% eines pharmaverträglichen Tensides oder Tensidgemisches

0 bis 10 Gewichts-% eines Vitamins oder Provitamins

0 bis 10 Gewichts-% einer freien Fettsäure und

übliche Trägerstoffe und/oder Verdünnungsmittel enthält.

4. Ein spontan dispergierbares Konzentrat gemäss Anspruch 3, dadurch gekennzeichnet, dass es als antitumorale Komponente 0.001 bis 15 Gewichts-% eines Sterol-Fettsäureesters der Formeln (I) bis (X) enthält, worin R eine Alkyl- oder Alkenylgruppe mit 8 bis 10 Kohlenstoffatomen und R' eine Alkyl- oder Alkenylgruppe mit 8 bis 22 Kohlenstoffatomen bedeuten.

5. Ein spontan dispergierbares Konzentrat gemäss Anspruch 4, dadurch gekennzeichnet, dass es als antitumorale Komponente 0.001 bis 15 Gewichts-% eines Sterol-Fettsäureesters der Formeln (I) bis (X) enthält, worin R eine Alkyl- oder Alkenylgruppe mit 8 bis 10 Kohlstoffatomen und R' eine Alkyl- oder Alkenylgruppe mit 10 bis 18 Kohlenstoffatomen bedeuten.

6. Ein spontan dispergierbares Konzentrat gemäss Anspruch 5, dadurch gekennzeichnet, dass es als antitumorale Komponente 0.001 bis 15 Gewichts-% eines der folgenden Sterol-Fettsäureester oder eines Gemisches derselben enthält:
STIGMASTEROL-10-UNDECENOAT
STIGMASTEROL-2-DODECENOAT
STIGMASTEROL-OLEAT
STIGMASTEROL-LINOLEAT
STIGMASTEROL-LINOLENAT
β-SITOSTEROL-10-UNDECENOAT
β-SITOSTEROL-2-DODECENOAT
β-SITOSTEROL-OLEAT
β-SITOSTEROL-LINOLEAT
β-SITOSTEROL-LINOLENAT
CHOLESTERYL-10-UNDECENOAT
CHOLESTERYL-2-DODECENOAT
CHOLESTERYL-OLEAT
CHOLESTERYL-LINOLEAT
CHOLESTERYL-LINOLENAT

7. Ein spontan dispergierbares Konzentrat gemäss Anspruch 2, dadurch gekennzeichnet, dass das pharmaverträgliche Tensid oder Tensidgemisch anionaktiv, kationaktiv, amphoter oder nicht-ionogen, vorzugsweise nichtionogen ist und ein hydrophiles-lipophiles Verhältnis zwischen 2 und 18, vorzugsweise zwischen 2 bis 6 einerseits und 10 bis 15 anderseits hat und das Hydrotrop, bzw. der Co-Emulgator ein aliphatischer Carbonsäureester wie Isopropyllaurat, Hexyllaurat, Decyllaurat, Isopropylmyristat und/oder Lauryllaurat ist.

8. Ein spontan dispergierbares Konzentrat gemäss Anspruch 3, dadurch gekennzeichnet, dass das pharmaverträgliche Tensld oder Tensidgemisch anionaktiv, kationaktiv, amphoter oder nicht-ionogen, vorzugsweise nichtionogen ist und ein hydrophiles-lipophiles Verhältnis zwischen 2 und 18, vorzugsweise zwischen 2 bis 6 einerseits und 10 bis 15 anderseits hat und das Hydrotrop, bzw. der Co-Emulgator ein aliphatischer Carbonsäureester wie Isopropyllaurat, Hexyllaurat, Decyllaurat, Isopropylmyristat und/oder Lauryllaurat ist.

9. Ein spontan dispergierbares Konzentratgemäss Anspruch 3, dadurch gekennzeichnet, dass es 7,5 bis 15 Gewichts-% eines oder mehrerer Fettsäureester von Sterolen der Formeln (I) bis (X), 0 - 40 Gewichts-% Isopropylmyristat, Ollvenoel, Sonnenblumenoel kalt gepresst und unbehandelt, Maiskeimoel, Welzenkeimoel oder Disteloel, 22,5 bis 42,5 Gewichts-% eines Mischemulgators, bestehend aus je 50 % der beiden Verbindungen mit Formeln

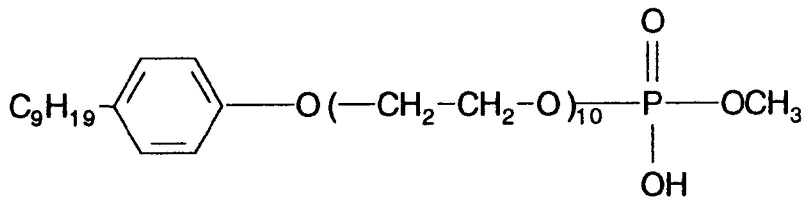

und

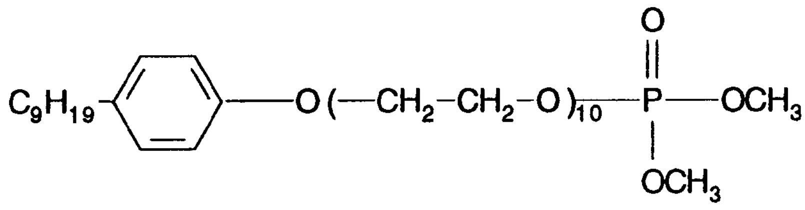

und 22,5 bis 45 Gewichts-% des tert. Octylphenylpolyoxyäthylenäthers mit 9 - 10 Oxyäthylengruppen enthält.

**10.** Ein spontan dispergierbares Konzentrat gemäss Anspruch 3, dadurch gekennzeichnet, dass es 7,5 bis 15 Gewichts-% eines oder mehrerer Fettsäureester von Sterolen der Formeln (I) bis (X), sowie 0 bis 40 Gewichts-% Isopropylmyristat, 22.5 bis 42,5 Gewichts-% eines tert. Octylphenolpolyoxyäthylenäthers mit 9 bis 10 Oxyäthylengruppen (Invadin® JFC 800%) und 22,5 bis 42,5 Gewichts-% eines äthoxylierten Tristyrylphenolphosphates mit Formel

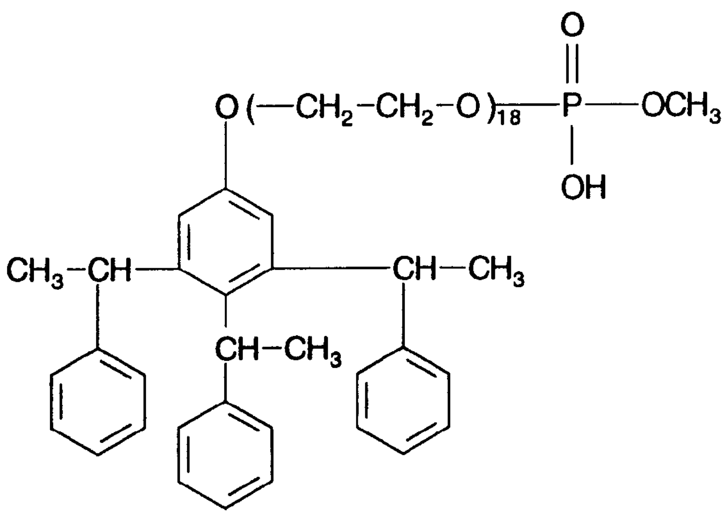

(Soprophor® FL bzw. FLK) enthält.

**11.** Ein spontan dispergierbares Konzentrat gemäss Anspruch 9, dadurch gekennzeichnet, dass es als Fettsäureester von Sterolen 7,5 bis 15 Gewichts-% β-Sitosterol-10-Undecenoat, β-Sitosterol-Lauroylat, β-Sitosterol-Palmitat, β-Sitosterol-Oleat, β-Sitosterol-Linoleat, β-Sitosterol-Linolenat, Stigmasterol-10-Undecenoat, Stigmasterol-Lauroylat, Stigmasterol-Palmitat, Stigmasterol-Oleat, Stigmasterol-Linoleat, Stigmasterol-Linolenat, Cholesteryl-10-Undecenoat, Cholesteryl-Lauroylat, Cholesteryl-Palmitat, Cholesteryl-Oleat, Cholesteryl-Linoleat, und/oder Cholesteryl-Linolenat enthält.

**12.** Ein spontan dispergierbares Konzentrat gemäss Anspruch 9, dadurch gekennzeichnet, dass es als Fettsäureester 7,5 bis 15 Gewichts-% Cholesteryl-2-Dodecenoat, β-Sitosterol-2-Dodecenoat und/oder Stigmasterol-2-Dodecenoat enthält.

**13.** Ein spontan dispergierbares Konzentrat gemäss Anspruch 10, dadurch gekennzeichnet, dass es als Fettsäureester 7,5 bis 15 Gewichts-% Cholesteryl-2-Dodecenoat, β-Sitosterol-2-Dodecenoat und/oder Stigmasterol-2-Dodecenoat enthält.

**14.** Die Sterol-Fettsäureester der Formeln

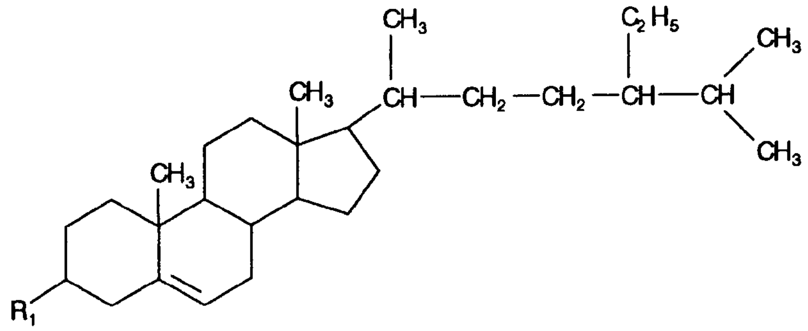

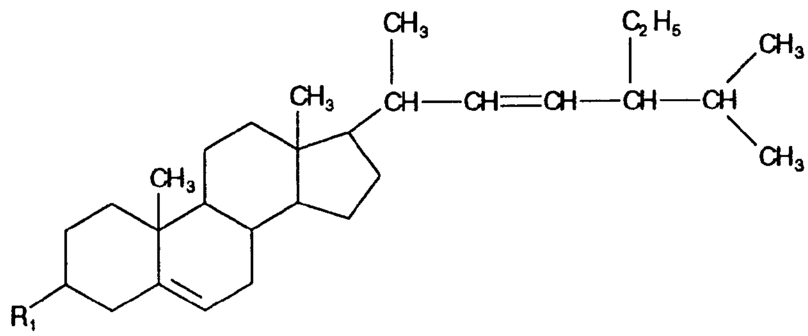

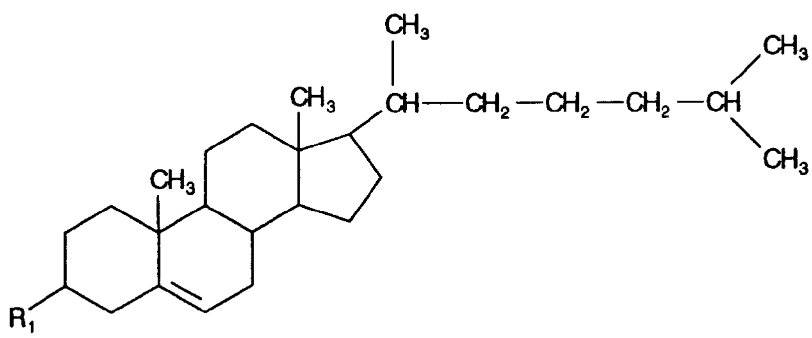

worin R1 den Rest

$CH_3$-$(CH_2)_4$-CH=CH-$(CH_2)_4$-COO-

bedeutet.

**15.** Ein Verfahren zur Herstellung der Sterol-Fettsäureester gemäss Anspruch 14, dadurch gekennzeichnet, dass man die Fettsäure der Formel

$CH_3$-$(CH_2)_4$ -CH=CH-$(CH_2)_4$-COO-

mit N,N'-Carbonyldiimidazol bei 25 bis 70 °C unter Zusatz einer katalytischen Menge eines Alkoholates in Tetrahydrofuran, Benzol, Chloroform oder einem ähnlichen indifferenten Lösungsmittel umsetzt und anschliessend das entstandene Fettsäureimidazolat mit einem Sterol laut folgenden Formeln

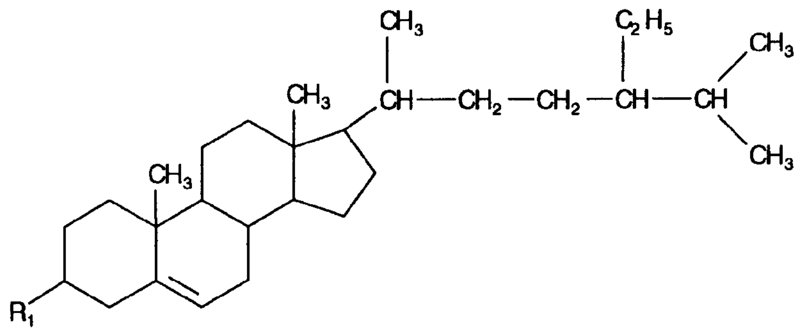

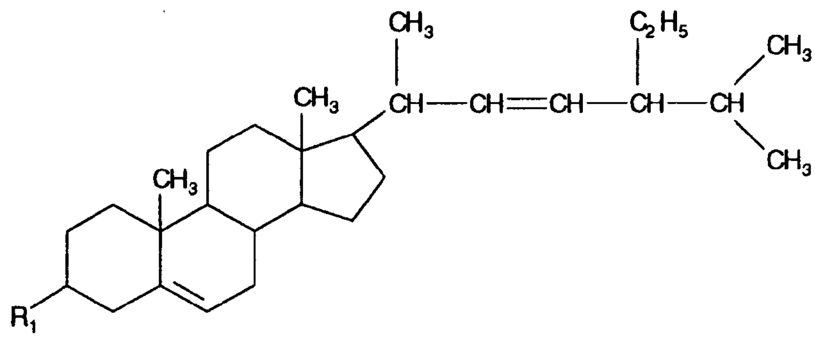

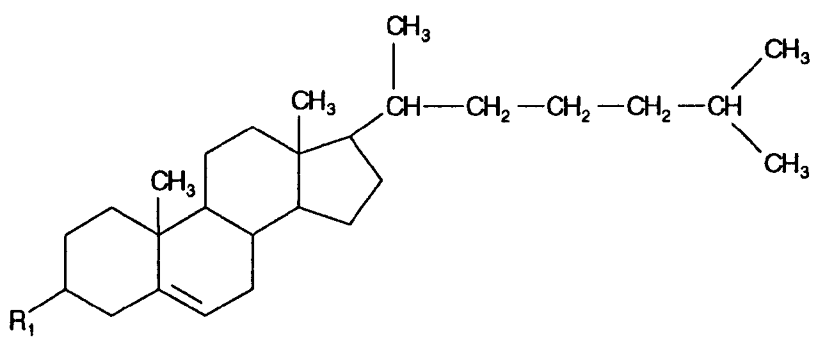

reagieren lässt.

## Claims

1. A process for obtaining the intercellular active substances from the seeds of plant species belonging to the compositae family (Compositae) and the gourd family (Cucurbitaceae), which comprises pregerminating the seeds for 2-4 days and then treating them with distilled water containing 0.1 - 4 % of manitol and 0.1 - 4 % of a pharmaceutically acceptable, non-ionic surfactant or surfactant mixture (relative to the weight of the pregerminated seeds); homogenizing the mixture thus obtained in a crosshead mill, followed by centrifugation; separating the resulting three phases from each other; discarding the bottom phase which contains husk fragments and cell debris; subjecting the upper, lipophilic phase to an extraction step and then purifying it by preparative chromatography, and subjecting the middle, aqueous phase first to ultrafiltration, then concentrating it in vacuo and iyophilizing it as a concentrate.

2. A spontaneously dispersible concentrate, which contains, as the antitumor component, 0.001 to 15 % by weight of the lipophilic phase prepared and purified as claimed in claim 1), and/or 0.001 to 30 % by weight of the lyophilized extract from the pregerminated seeds of Helianthus annuus L., Cucurbita pepo L. or Cucurbita maxima Duch., prepared as claimed in claim 1), and also 0.001 to 20 % by weight of a pharmaceutically acceptable surfactant or surfactant mixture, 0.001 to 40 % by weight of a pharmaceutically acceptable solvent or solvent mixture which serves as a hydrotropic agent or coemulsifier, and, if appropriate, customary excipients and/or diluents or stabilizers.

3. A spontaneously dispersible concentrate which contains as the antitumor component 0.001 to 15 % by weight of a fatty acid ester of a sterol, or a combination of such esters, with formulae:

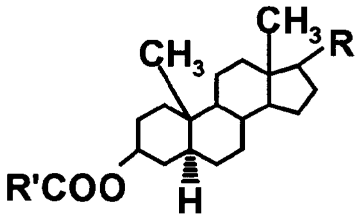

(I)

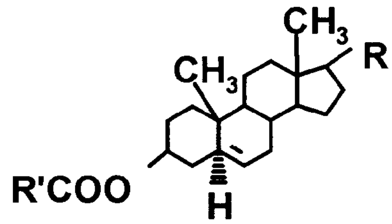

(II)

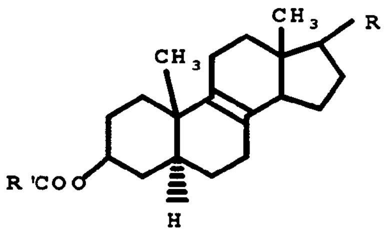

(III)

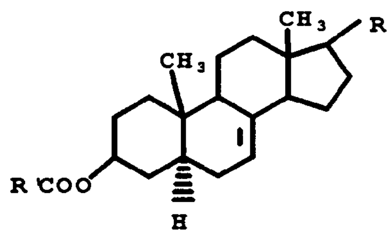

(IV)

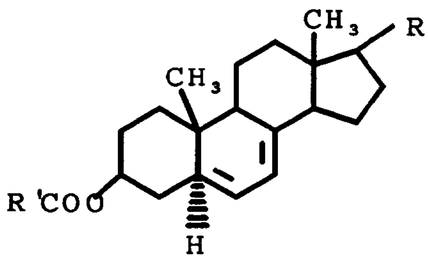

(V)

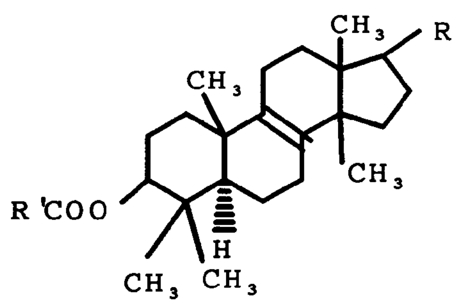

(VI)

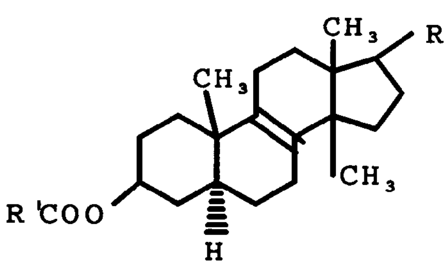

(VII)

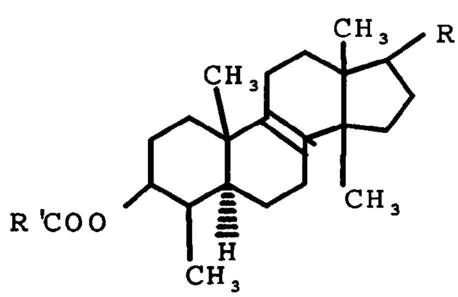

(VIII)

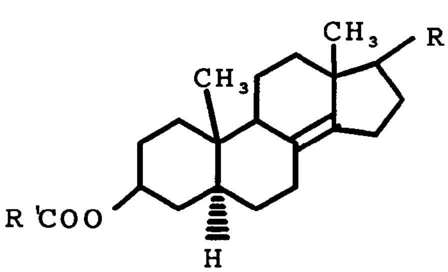

(IX)

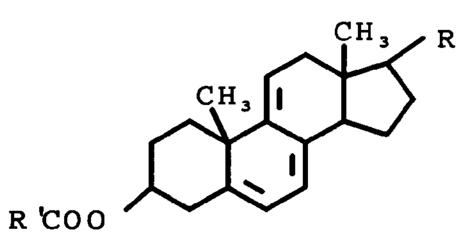

(X)

in which R represents a $C_1$ to $C_{10}$ alkyl or a $C_2$ to $C_{10}$ alkenyl group and R' stands for a $C_1$ to $C_{32}$ alkyl or

a $C_2$ to $C_{32}$ alkenyl group, as well as
0 to 40 % by weight of a pharmaceutically acceptable solvent or sol-vent mixture which serves as a hydrotropic agent or coemulsifier
0 to 10 % by weight of a vitamin or provitamin
0 to 10 % of a free fatty acid and
customary auxiliaries and/or diluents.

4. A spontaneously dispersible concentrate according to claim 3) which contains as the antitumor component 0.001 to 15 % by weight of a fatty acid steryl ester of the formulae (I) to (X), in which R means a $C_8$ to $C_{10}$ alkyl or alkenyl group and R' stands for a $C_8$ to $C_{22}$ alkyl or alkenyl group.

5. A spontaneously dispersible concentrate according to claim 4) which contains as the antitumor component 0.001 to 15 % by weight of a fatty acid steryl ester of the formulae (I) to (X), in which R means a $C_8$ to $C_{10}$ alkyl or alkenyl group and R' stands for a $C_{10}$ to $C_{18}$ alkyl or alkenyl group.

6. A spontaneously dispersible concentrate according to claim 5) which contains as the antitumor component 0.001 to 15 % by weight of one or several in combination of the following fatty acid steryl esters
STIGMASTERYL-10-UNDECENOATE
STIGMASTERYL-2-DODECENOATE
STIGMASTERYL-OLEATE
STIGMASTERYL-LINOLEATE
STIGMASTERYL-LINOLENATE
β-SITOSTERYL-10-UNDECENOATE
β-SITOSTERYL-2-DODECENOATE
β-SITOSTERYL-OLEATE
β-SITOSTERYL-LINOLEATE
β-SITOSTERYL-LINOLENATE
CHOLESTERYL-10-UNDECENOATE
CHOLESTERYL-2-DODECENOATE
CHOLESTERYL-OLEATE
CHOLESTERYL-LINOLEATE
CHOLESTERYL-LINOLENATE

7. A spontaenously dispersible concentrate as claimed in claim 2), wherein the pharmaceutically acceptable surfactant or surfactant mixture is anionic, cationic, amphoteric or non-ionic, preferably nonionic, and has a hydrophilic-lipophilic balance of between 2 and 18, preferably of between 2 and 6 on the one hand and 10 to 15 on the other hand, and the hydrotropic agent or the coemulsifier is an aliphatic carboxylic acid, such as isopropyl laurate, hexyl laurate, decyl laurate, isopropyl myristate and/or lauryl laurate.

8. A spontaenously dispersible concentrate as claimed in claim 3), wherein the pharmaceutically acceptable surfactant or surfactant mixture is anionic, cationic, amphoteric or non-ionic, preferably nonionic, and has a hydrophilic-lipophilic balance of between 2 and 18, preferably of between 2 and 6 on the one hand and 10 to 15 on the other hand, and the hydrotropic agent or the coemulsifier is an aliphatic carboxylic acid, such as isopropyl laurate, hexyl laurate, decyl laurate, isopropyl myristate and/or lauryl laurate.

9. A spontaneously dispersible concentrate as claimed in claim 3), which contains 7.5 to 15 % by weight of one or a combination of several fatty acid steryl esters according to formulae (I) to (X), 0 to 40 % by weight isopropyl myristate, olive oil, cold pressed, untreated sunflower or carthamus seed oil, corn germ oil, or wheat germ oil, 22.5 to 42.5 % by weight of a 50:50 mixture of the two tensides with formulae

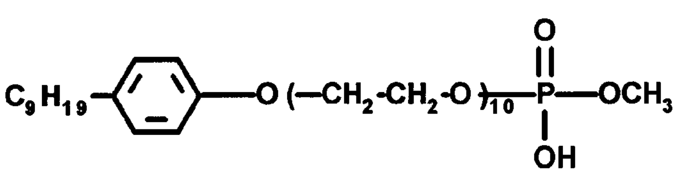

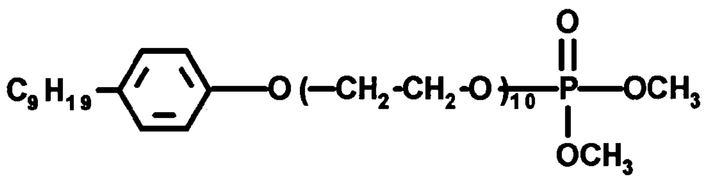

(Diphasol®, CIBA-GEIGY)
and 22.5 to 45 % by weight of the tert. octylphenylpolyoxyethyleneether with 9 to 10 oxyethylene groups (INVADIN® JFC 800%).

**10.** A spontaneously dispersible concentrate according to claim 3), which contains 7,5 to 15 % by weight of one or a combination made up of several fatty acid steryl ester compounds of the formulae (I) to (X) as well as 0 to 40 % by weight isopropylmyristate, 22.5 to 42.5 % of the tert. octylphenylpolyoxyethyleneether with 9 to 10 oxyethylene groups (Invadin® JFC 800%) and 22.5 to 42.5 % by weight of the ethoxy-lated tristyrylphenolphosphate with formula

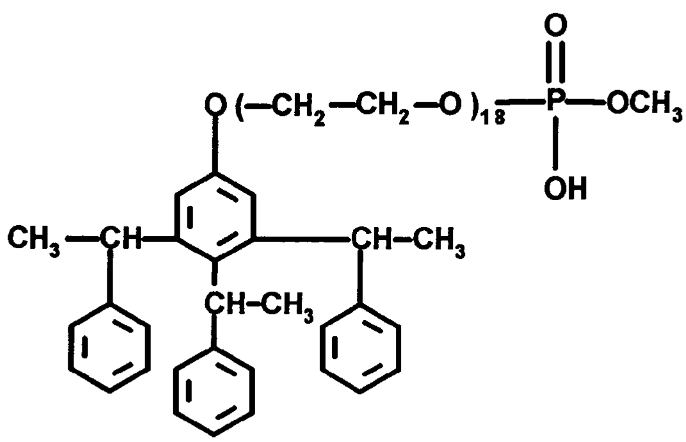

(Soprophor® FL, Rhône-Poulenc).

**11.** A spontaneously dispersible concentrate as claimed in claim 9), which contains 7.5 to 15 % by weight of the fatty acid steryl esters β-Sitosteryl-10-undecenoate, β-Sitosteryl-laurate, β-Sitosteryl-palmitate, β-Sitosteryl-oleate, β-Sitosteryl-linoleate, β-Sitosteryl-linolenate, Stigmasteryl-10-undecenoate, Stigmasteryl-laurate, Stigmasteryl-palmitate, Stigmasteryl-oleate, Stigmasteryl-linoleate, Stigmasteryl-linolenate, Cholesteryl-10-undecenoate, Cholesteryl-laurate, Cholesteryl-palmitate, Cholesteryl-oleate, Cholesteryl-linoleate, and/or Cholesteryl-linolenate,

**12.** A spontaneously dispersible concentrate as claimed in claim 9), which contains 7.5 to 15 % by weight of the fatty acid steryl esters Cholesteryl-2-dodecenoate, β-Sitosteryl-2-dodecenoate and/or Stigmasteryl-2-dodecenoate.

**13.** A spontaneously dispersible concentrate as claimed in claim 10), which contains 7.5 to 15 % by weight of the fatty acid steryl esters Cholesteryl-2-dodecenoate, β-Sitosteryl-2-dodecenoate and/or Stigmasteryl-2-dodecenoate.

**14.** The fatty acid steryl esters of formulae

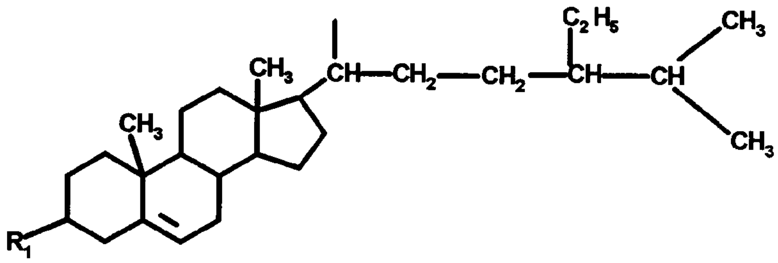

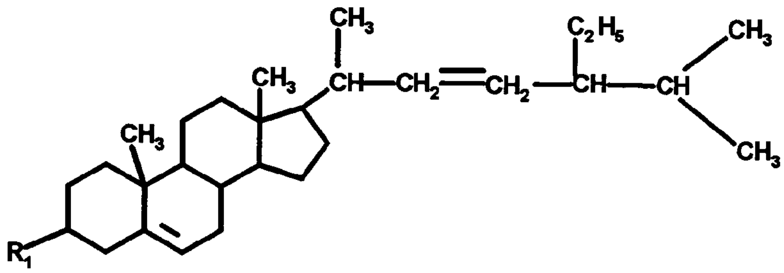

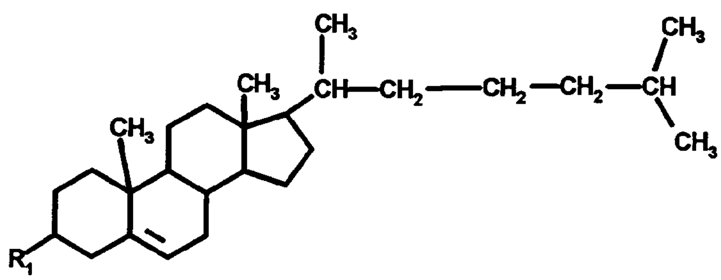

wherein $R_1$ signifies the radical

$$CH_3\text{-}(CH_2)_4\text{-}CH{=}CH\text{-}(CH_2)_4\text{-}COO\text{-}$$

**15.** A procedure for the preparation of fatty acid steryl esters according to claim 14) whereby the fatty acid of formula

$$CH_3\text{-}(CH_2)_4\text{-}CH{=}CH\text{-}(CH_2)_4\text{-}COO\text{-}$$

is first reacted with N,N'carbonyldiimidazole at 25 to 70 °C under addition of a catalytic amount of an alcoholate in tetrahydrofurane, benzene, chloroform or a similar indifferent solvent, with subsequent reaction of the formed fatty acid imidazolate with a sterol of formulae

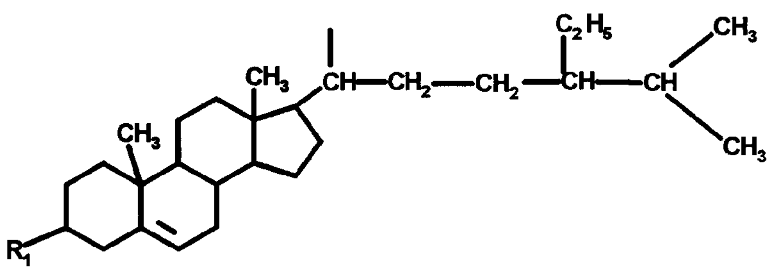

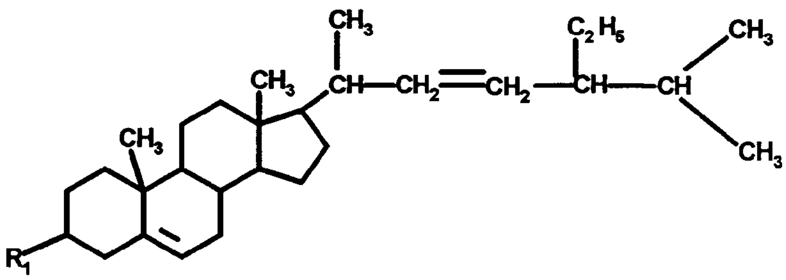

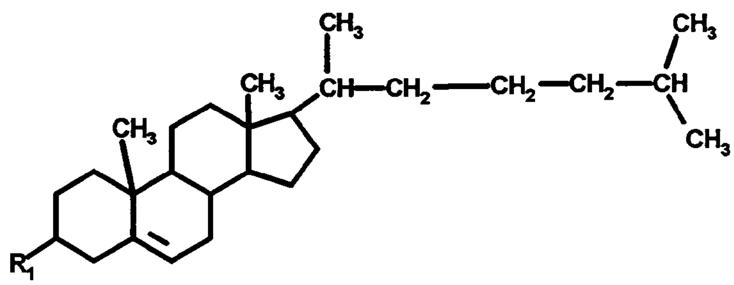

.

## Revendications

1. Un procédé pour obtenir les substances actives intercellulaires des semences de plantes qui appartiennent à la famille des Compositae et à la famille des Cucurbitaceae qui comprend les stades suivants: prégermination des semences pendant 2-4 jours, traitement avec de l'eau distillée contentant 0,1 - 4 % de manitol et 0,1 - 4 % d'un surfactant ou d'une combinaison de surfactants non-ioniques, acceptés en pharmacie (poids relatif aux semences pregermés); homogénisation du mélange avec un moulin à tête croisée; séparation des trois phases qui résultent et élimination de la phase lourde qui contient les gaines et des débris de cellules; extraction de la phase supérieure, lipophilique et purification sur colonne de chromatogaphie préparative; ultrafiltration de la phase moyenne, aqueuse, avec concentration subséquente dans le vide et lyophilisation comme concentré.

2. Un concentré spontanément dispersible qui contient comme composé antitumoral, 0.001 à 15 % par poids de la phase lipophilique préparée et purifiée selon revendication 1), et/ou 0.001 à 30 % par poids de l'extrait lyophilisé obtenu des semences prégermés de Helianthus annuus L., Cucurbita pepo L. ou Cucurbita maxima Duch., préparé en accord avec revendication 1), ainso que 0.001 à 20 % par poids d'un surfactant ou d'une combinaison de surfactants acceptés en pharmacie, 0.001 à 40 % par poids d'un solvent ou d'une combinaison de solvents qui servent comme agent hydrotropique ou coémulsifiant, et, si indiqué, des excipients, diluants ou stabilisateurs habituels.

3. Un concentré spontanément dispersible qui contient comme composé antitumoral, 0.001 à 15 % par poids d'un ester d'un acide gras formé avec un sterol, ou d'une combinaison de tels esters, avec formule:

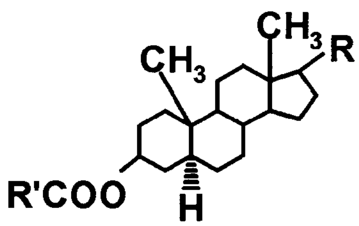

(I)

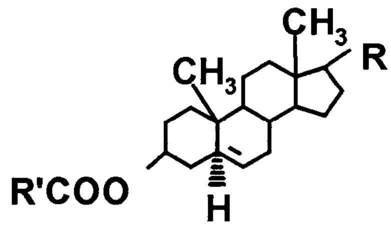

(II)

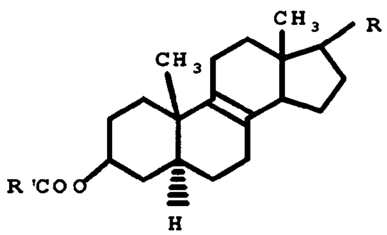

(III)

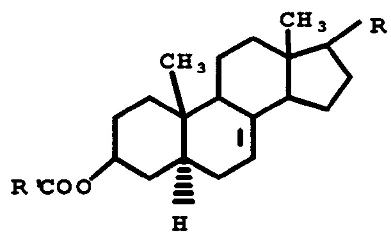

(IV)

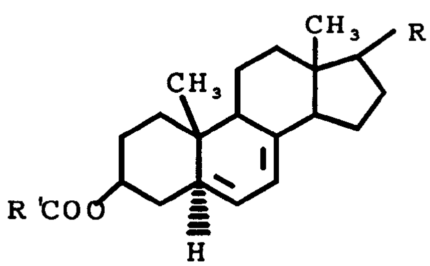

(V)

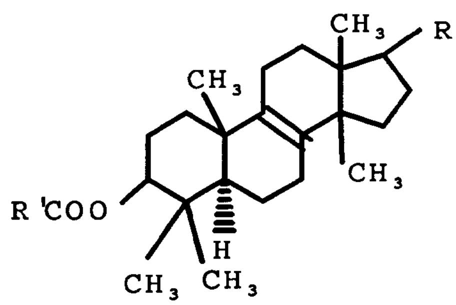

(VI)

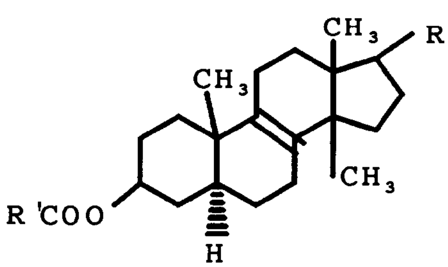

(VII)

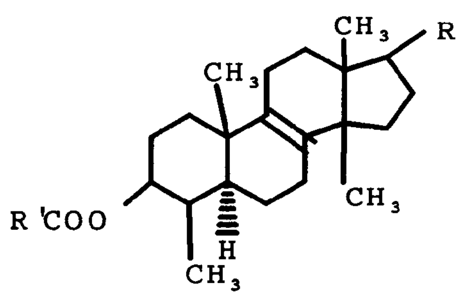

(VIII)

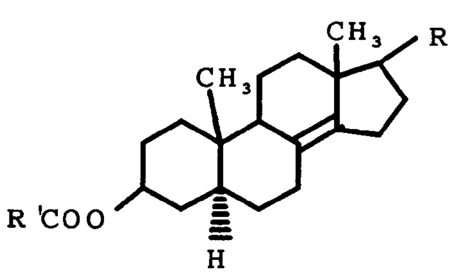

(IX)

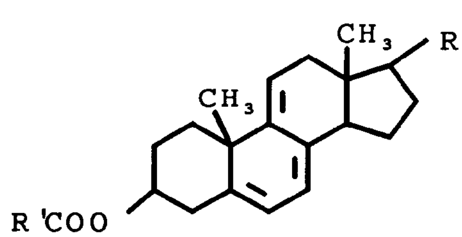

(X)

dans lesquelles R représente un groupe $C_1$ à $C_{10}$ alkyl ou $C_2$ à $C_{10}$ alkenyl et R' signifie un groupe $C_1$ à $C_{32}$ alkyl ou $C_2$ à $C_{32}$ alkenyl, ainsi que

0 à 40 % par poids d'un solvent ou d'une combinaison de solvants acceptés en pharmacie qui servent d'agent hydrotropique ou comme coémulsifiant
0 à 10 % par poids d'une vitamine ou provitamine
0 à 10 % par poids d'un acide gras libre et
des auxiliaires et/ou diluants habituels.

**4.** Un concentré spontanément dispersible conforme à la revendication 3) qui contient come composé antitumoral, 0.001 à 15 % par poids d'un ester d'un acide gras formé avec un sterol, ou une combinaison de tels esters, selon formules (I) à (X), dans lesquelles R représente un groupe $C_8$ à $C_{10}$ alkyle ou alkenyle et R' désigne un groupe $C_8$ à $C_{22}$ alkyle ou alkenyle.

**5.** Un concentré spontanément dispersible conforme à la revendication 3) qui contient come composé antitumoral, 0.001 à 15 % par poids d'un ester d'un acide gras formé avec un sterol, ou une combinaison de tels esters, selon formules (I) à (X), dans lesquelles R représente un groupe $C_8$ à $C_{10}$ alkyle ou alkenyle et R' désigne un groupe $C_{10}$ à $C_{18}$ alkyle ou alkenyle.

**6.** Un concentré spontanément dispersible conforme à la revendication 5) qui contient, comme composé antitumoral, 0.001 à 15 % par poids de l'un ou plusieurs des esters formés avec das acides gras énoncés ci-après

STIGMASTERYL-10-UNDECENOAT
STIGMASTERYL-2-DODECENOAT
STIGMASTERYL-OLEAT
STIGMASTERYL-LINOLEAT
STIGMASTERYL-LINOLENAT
β-SITOSTERYL-10-UNDECENOAT
β-SITOSTERYL-2-DODECENOAT
β-SITOSTERYL-OLEAT
β-SITOSTERYL-LINOLEAT
β-SITOSTERYL-LINOLENAT
CHOLESTERYL-10-UNDECENOAT
CHOLESTERYL-2-DODECENOAT
CHOLESTERYL-OLEAT
CHOLESTERYL-LINOLEAT
CHOLESTERYL-LINOLENAT

**7.** Un concentré spontanément dispersible, conforme à la revendication 2), dans lequel le surfactant et la combinaison de surfactants acceptés en pharmacie sont anionique, cationique, amphotérique ou non-ionique et possèdent un équilibre lipophilique-hydrophilique entre 2 et 18, de préférence entre 2 et 6 d'un côté et 10 et 15 de l'autre et ou l'agent hydrotropique ou coémulgateur est un acide aliphatique carboxylique, comme le laurate d'isopropyle, le laurate de hexyle, le laurate de décyle, le myristate d'isopropyle et/ou le laurate de lauryle.

**8.** Un concentré spontanément dispersible, conforme à la revendication 3), dans lequel le surfactant et la combinaison de surfactants acceptés en pharmacie sont anionique, cationique, amphotérique ou non-ionique et possèdent un équilibre lipophilique-hydrophilique entre 2 et 18, de préférence entre 2 et 6 d'un côté et 10 et 15 de l'autre et ou l'agent hydrotropique ou coémulgateur est un acide aliphatique carboxylique, comme le laurate d'isopropyle, le laurate de hexyle, le laurate de décyle, le myristate d'isopropyle et/ou le laurate de lauryle.

**9.** Un concentré spontanément dispersible conforme à la revendication 3), qui contient 7.5 à 15 % par poids de l'un ou de plusieurs esters de sterols formés avec des acides gras selon les formules (I) à (X), 0 à 40 % par poids de myristate d'isopropyle, d'huile d'olives pressé à froid, non traité, d'huile de semences de chardon, de germes de maïs ou de germes de froment, ainsi que 22.5 à 42,5 % par poids d'un mélange 50:50 des surfactants avec formules

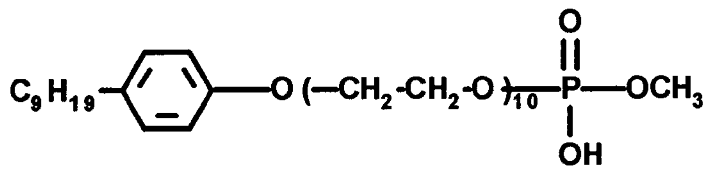

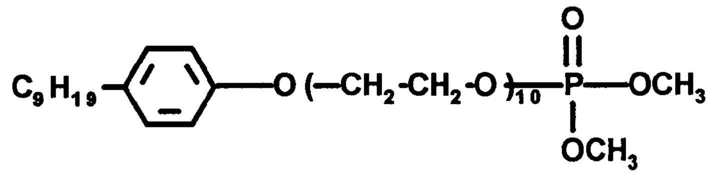

(Diphasol®, CIBA-GEIGY)
et 22.5 à 45 % par poids de l'éther tert. de octylphénylpolyoxyéthylène avec 9 à 10 groupes oxyéthylène (INVADIN® JFC 800%).

**10.** Un concentré spontanément dispersible conforme à la revendication 3), qui contient 7.5 à 15 % par poids de l'un ou de plusieurs esters de sterols formés avec des acides gras en accord avec les formules (I) à (X), 0 à 40 % par poids de myristate d'isopropyle, 22,5 à 42.5 % de l'éther tert. de octylphénylpolyoxyéthylène avec 9 à 10 groupes oxyéthylène (INVADIN® JFC 800%). ainsi que 22.5 à 42.5 % du phosphate tristyrylphénole éthoxylé, avec formule

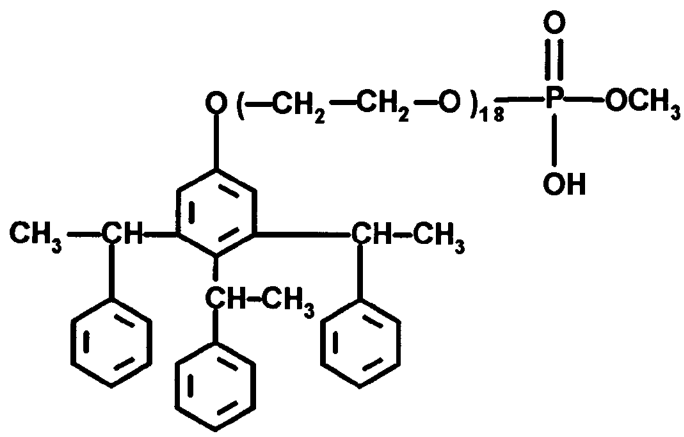

(Soprophor® FL, Rhône-Poulenc).

**11.** Un concentré spontanément dispersible conforme à la revendication 9), qui contient 7.5 à 15 % par poids des esters de sterols formés avec un acide gras suivants: β-Sitosteryl-10-undécenoat, β-Sitosteryl-laurat, β-Sitosteryl-palmitat, β-Sitosteryl-oléat, β-Sitosteryl-linoléat, β-Sitosteryl-linolénat, Stigmasteryl-10-undécenoat, Stigmasteryl-laurat, Stigmasteryl-palmitat, Stigmasteryl-oléat, Stigmasteryl-linoléat, Stigmasteryl-linolénat, Cholesteryl-10-undécenoat, Cholesteryl-laurat, Cholesteryl-palmitat, Cholesteryl-oléat, Cholesteryl-linoléat et/ou Cholesteryl-linolénat,

**12.** Un concentré spontanément dispersible conforme à la revendication 9), qui contient 7.5 à 15 % par poids un des esters de sterols formés avec un acide gras suivants: Cholesteryl-2-dodécenoat, β-Sitosteryl-2-dodécenoat et/ou Stigmasteryl-2-dodécenoat.

**13.** Un concentré spontanément dispersible conforme à la revendication 10) qui contient 7.5 à 15 % par poids un des esters de sterols formés avec un acide gras suivants: Cholesteryl-2-dodécenoat, β-Sitosteryl-2-dodécenoat et/ou Stigmasteryl-2-dodécenoat.

**14.** Les esters de sterols formés avec un acide gras de formules

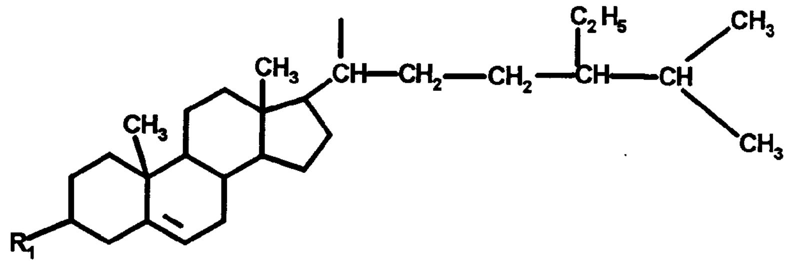

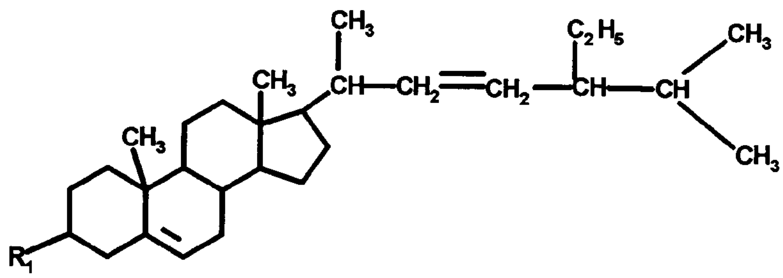

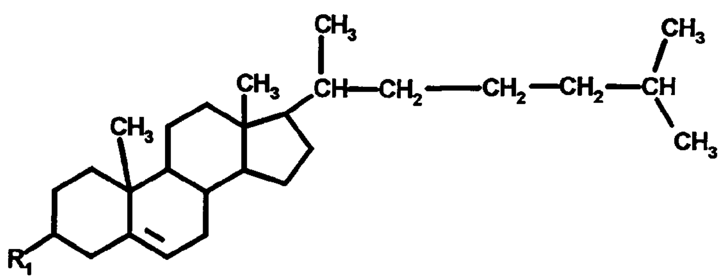

dans lesquelles $R_1$ signifie le radical

$$CH_3-(CH_2)_4-CH=CH-(CH_2)_4-COO-$$

**15.** Un procédé de préparation d'esters de sterols avec des acides gras en accord avec la revendication 14), ou l'acide gras de formule

$$CH_3-(CH_2)_4-CH = CH -(CH_2)_4-COO-$$

est d'abord réagi avec du N,N'carbonyldiimidazol à une température de 25 à 70 °C sous addition d'un montant catalytique d'un alcoholat dans du tetrahydrofurane, benzène, chloroforme ou un solvent indifférent analogue, et est suivi par la réaction de l'imidazolat formé avec l'acide gras, avec un composé de sterol de formule

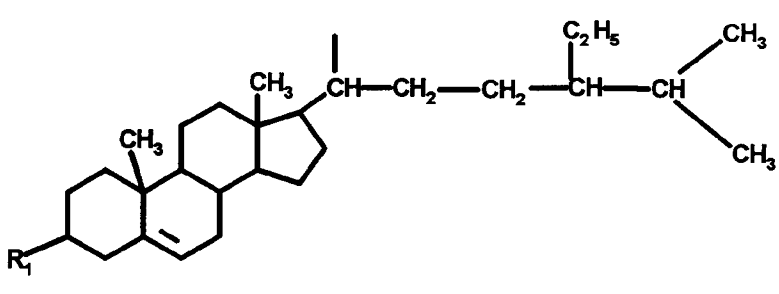

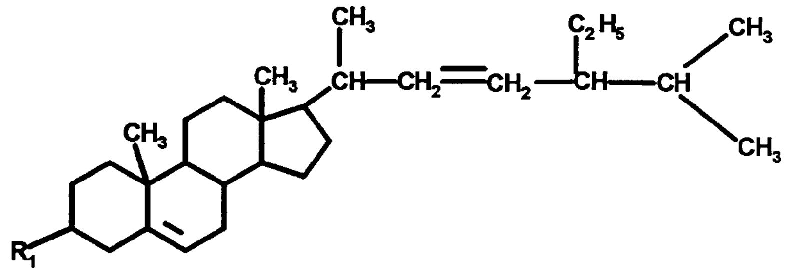

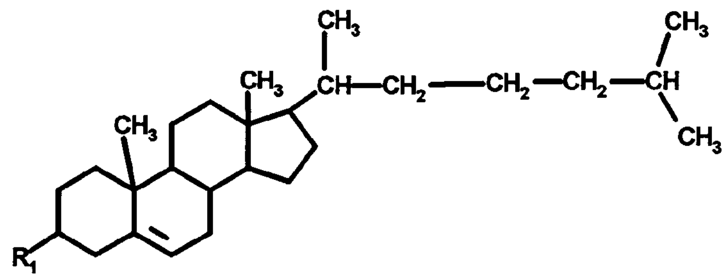

.

## S C H M E L Z P U N K T E

Gemessen mit einem Mettler-Gerät TA 4000 DSC-Verfahren (Differential Scanning Calorimetry) Rate: 2.5°C/min., bzw. mit einem Büchi-Gerät No. 535

| MESSWERT VERBINDUNG | ONSET (Smp) | PEAK ° C | ENDOTHERME REAKTION |
|---|---|---|---|
| C 8:0 CHOLESTEROL | 106,6 | 107,6 | 103 - 110 |
| C 8:0 SITOSTEROL | 71,3 | 72,5 | 64 - 76 |
| C 8:0 STIGMASTEROL | 89,9 | | 89,5 - 90,4 |
| C 11:1 CHOLESTEROL | 78,9 | 79,7 | 78 - 88 |
| C 11:1 SITOSTEROL | 68,3 | | |
| C 11:1 STIGMASTEROL | 85,7 | 86,7 | 80 - 90 |
| C 12:0 CHOLESTEROL | 79,4 | 79,9 | 76 - 82 |
| C 12:0 SITOSTEROL | 82,4 | 83,3 | 78 - 88 |
| C 12:0 STIGMASTEROL | 97,1 | 98,0 | 90 - 101 |
| C 16:0 CHOLESTEROL | 77 - 79 | | |
| C 16:0 SITOSTEROL | 89,6 | 91,0 | 82 - 95 |
| C 16:0 STIGMASTEROL | 99,7 | 101,3 | 92 - 105 |
| C 18:2 CHOLESTEROL | 62 | | |
| C 18:3 CHOLESTEROL | 35 - 36 | | |
| STIGMASTEROL-ß-d-GLUCOSID | 255,9 - 256,7 | | |

## B R E C H U N G S I N D E X

Gemessen mit einem Zeiss-Refraktometer

| VERBINDUNG | MESSWERT $n_D$ 20°C |
|---|---|
| C 12:1 CHOLESTEROL | 1,4807 |
| C 12:1 SITOSTEROL | 1,5118 |
| C 12:1 STIGMASTEROL | 1,4913 |
| C 18:1 SITOSTEROL | 1,4900 |
| C 18:1 STIGMASTEROL | 1,4900 |
| C 18:2 SITOSTEROL | 1,4866 |
| C 20:4 SITOSTEROL | 1,4950 |

# Density Scanning Calorimetry

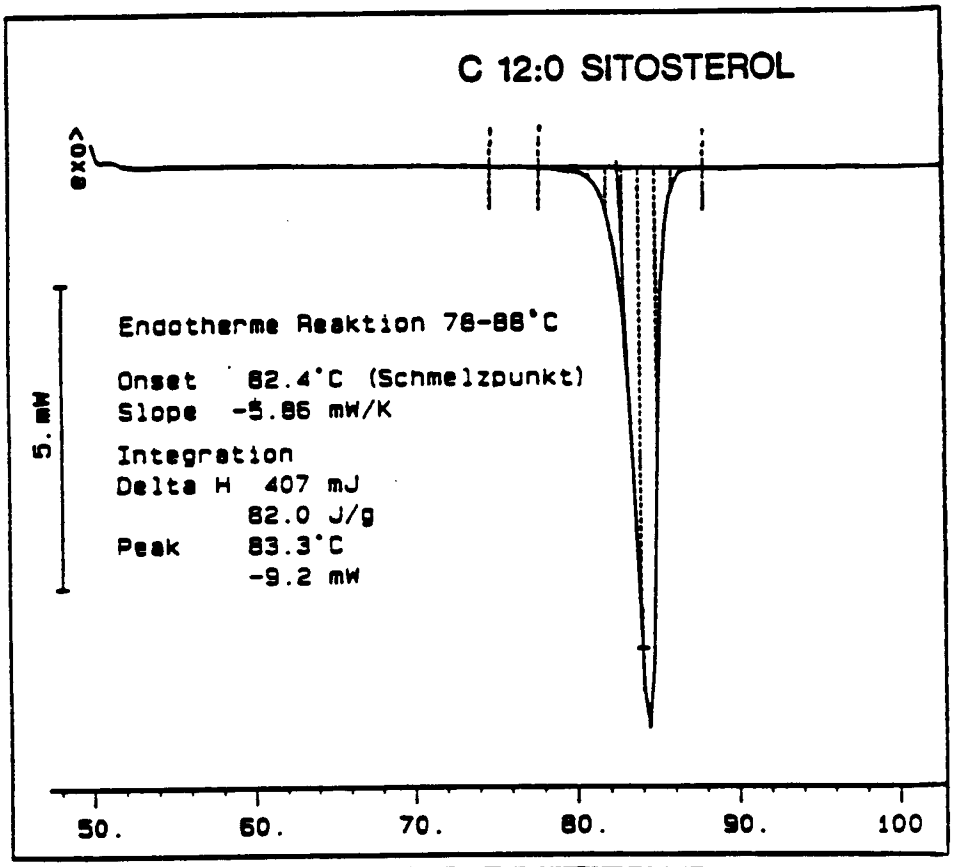


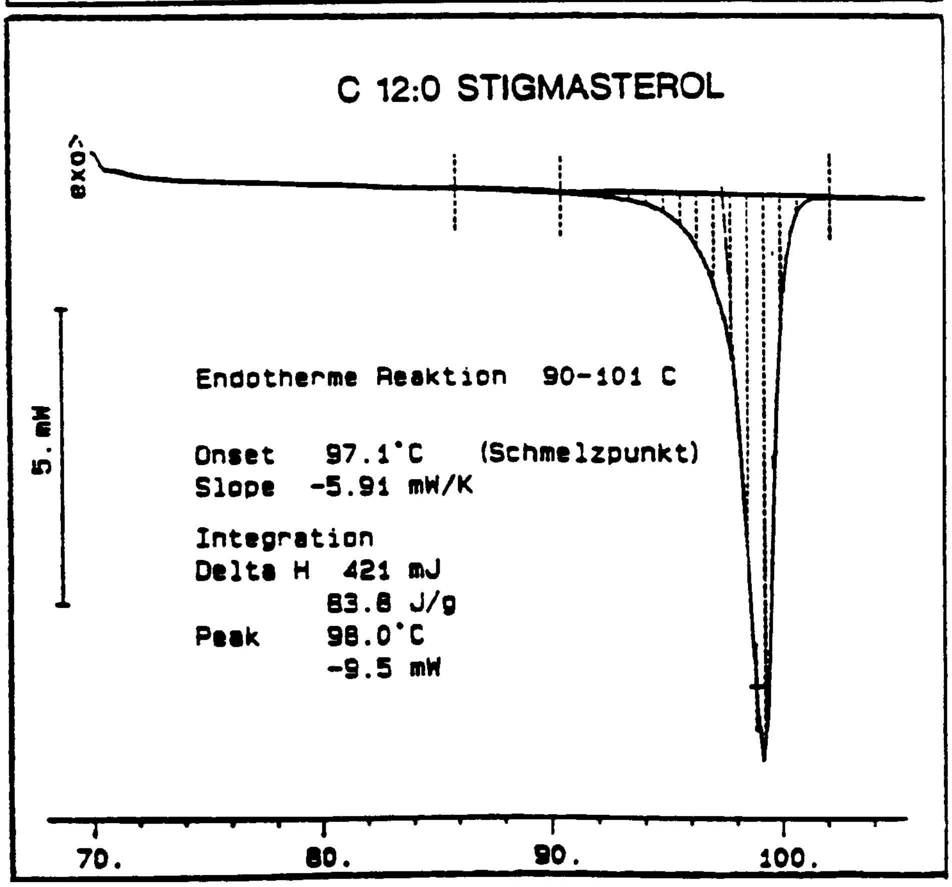

# Rf - W E R T E

1%-Lösung in $CH_2Cl_2$, bandförmig aufgetragen 2 cm/2µl
LINOMAT III CAMAG 10 cm run
UV 366 nach 1:1 $H_2SO_4$/MeOH 2 min 120° C

| SYSTEM / VERBINDUNG | 1 | 2 | 3 | 4 | 5 | 6 | 7 |
|---|---|---|---|---|---|---|---|
| C 8:0 CHOL | 0,12 | 0,19 | 0,30 | 0,85 | 0.98 | 0,85 | 0,68 |
| C 11:1 CHOL | 0,25 | 0,30 | 0,43 | 0,80 | 0,96 | 0,83 | 0,72 |
| C 11:1 SITO | 0,24 | 0,25 | 0,35 | 0,89 | 0,91 | 0,81 | 0,67 |
| C 11:1 STIGMA | 0,23 | 0,24 | 0,35 | 0,88 | 0,90 | 0,80 | 0,65 |
| C 12:0 CHOL | 0,14 | 0,20 | 0,32 | 0,89 | 0,99 | 0,90 | 0,73 |
| C 12:0 SITO | 0,26 | 0,27 | 0,39 | 0,92 | 0,95 | 0,84 | 0,73 |
| C 12:0 STIGMA | 0,26 | 0,27 | 0,39 | 0,92 | 0,95 | 0,84 | 0,73 |
| C 12:1 CHOL | 0,56 | 0,60 | 0,58 | 0,98 | 0,95 | 0,96 | 0,95 |
| C 12:1 SITO | 0,56 | 0,60 | 0,58 | 0,98 | 0,95 | 0,96 | 0,95 |
| C 12:1 STIGMA | 0,56 | 0,60 | 0,58 | 0,98 | 0,95 | 0,96 | 0,95 |
| C 16:0 SITO | 0,28 | 0,29 | 0,41 | 0,93 | 0,97 | 0,87 | 0,77 |
| C 16:0 STIGMA | 0,28 | 0,29 | 0,41 | 0,93 | 0,97 | 0,86 | 0,78 |
| C 18:2 SITO | 0,65 | 0,65 | 0,64 | 0,95 | 0,98 | 0,97 | 0,94 |
| C 18:2 STIGMA | 0,65 | 0,65 | 0,64 | 0,95 | 0,98 | 0,98 | 0,94 |
| C 18:3 SITO | 0,65 | 0,65 | 0,64 | 0,94 | 0,97 | 0,97 | 0,94 |
| C 18:3 STIGMA | 0,65 | 0,65 | 0,64 | 0,94 | 0,98 | 0,98 | 0,93 |

**Erklärung:**

System 1 Platte Merck Art. 5715 Petrolether/Diethylether 98:2

System 2 do. do. 97:3

System 3 do. Cyclohexan/Ethylacetat 97:3

System 4 Platte Macherey Nagel RP 18 Art. 811'071 Petrolether/Diethylether 95:5

System 5 do. n-Hexan/t.Butylmethylether/Aceton 90:5:5

System 6 do. Petrolether/Cyclohexan/Ethylacetat/$H_2O$ 48:48:3:1

System 7 do. Petrolether/Diethylether 97:3

# H P L C - ANALYSE

Säule 25 cm/4,6 mm mit Nucleosil 5C18 300 A°
Eluiermittel: 100 % $CH_3CN$ + 0,1 % TFA R.T.
1 ml/Min. ca. 200 bar
Detektor U V = 200 nm

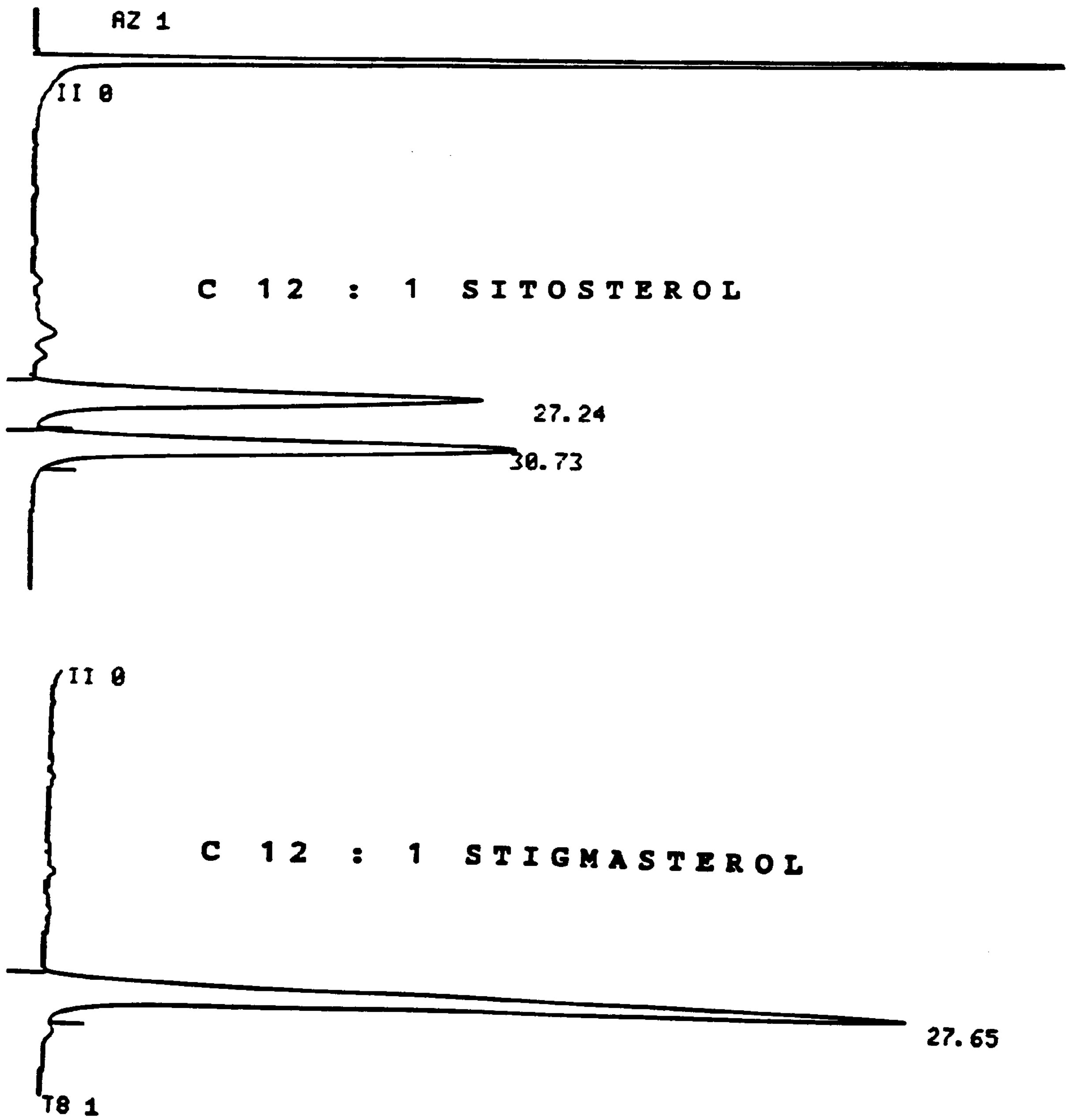

## S T I G M A S T E R O L - β - d - G L U C O S I D

## R f - W E R T E

**Auftrag 2 mg/1 ml = 0,2 % Lösung in $CHCl_3$ : MeOH : $H_2O$**
**70 : 30 : 5**
**im Wasserbad auf 60 °C anwärmen**

| | | |
|---|---|---|
| **System 1** | **Rf.** | **0,65** |
| **System 2** | **Rf.** | **0,56** |
| **System 3** | **Rf.** | **0,49** |
| **System 4** | **Rf.** | **0,60** |

**Erläuterung:**

| | | | | | |
|---|---|---|---|---|---|
| **System 1:** | $CHCl_3$ : | MeOH : | 17%$NH_3$ | | |
| | 41 | 41 | 18 | | |
| **System 2:** | $CHCl_3$ : | MeOH : | $H_2O$ | | |
| | 70 | 30 | 5 | | |
| **System 3:** | $CHCl_3$ : | MeOH : | $H_2O$ : | Essigsäure | |
| | 75 | 25 | 5 | 0,5 | |
| **System 4:** | n-Butanol : | $CCl_4$ : | MeOH : | Ameisensäure : | $H_2O$ |
| | 30 | 40 | 20 | 5 | 5 |

**D S C**

**DENSITY SCANNING CALORIMETRY**

**Mettler Gerät TA 4000 Rate 2,5 °C/min.**

**STIGMASTEROL-β-d-GLUCOSID**